# FASCICULE DE BREVET EUROPEEN

(11) **EP 2 342 329 B1**
(45) Date de publication et mention de la délivrance du brevet: **09.11.2016**
(21) Numéro de dépôt: 09741395.9
(22) Date de dépôt: 10.09.2009
(51) Int. Cl.: C12N 9/42, C12N 9/24, C12N 15/81

(54) **VARIANTS DE BETA-GLUCOSIDASE A ACTIVITE AMELIOREE ET LEURS UTILISATIONS**
BETA-GLUCOSIDASE-VARIANTEN MIT VERBESSERTER AKTIVITÄT UND VERWENDUNGEN DAVON
BETA-GLUCOSIDASE VARIANTS HAVING IMPROVED ACTIVITY, AND USES THEREOF

(30) Priorité: 12.09.2008 FR 0856167
(43) Date de publication de la demande: 13.07.2011
(73) Titulaire: IFP Énergies nouvelles, 92852 Rueil-Malmaison Cedex (FR); Proteus, 30000 Nîmes (FR)
(72) Inventeur: LOPES-FERREIRA, Nicolas, F-28210 Croisilles (FR); MARGEOT, Antoine, F-75020 Paris (FR); MATHIS, Hugues, F-77600 Bussy Saint Georges (FR); FOURAGE, Laurent, F-30420 Calvisson (FR)
(74) Mandataire: Cabinet Plasseraud
(86) Numéro de dépôt international: PCT/FR2009/051701
(87) Numéro de publication internationale: WO 2010/029259

(56) Documents cités:
- WO-A-2005/074647
- US-A1- 2004 253 702
- US-A1- 2007 077 630
- DATABASE Geneseq [Online] 17 juin 2004 (2004-06-17), "Trichoderma sp. C-4 beta-glucosidase, SEQ ID 2." XP002510451 extrait de EBI accession no. GSP:ADM94196 Database accession no. ADM94196
- DATABASE UniProt [Online] 20 mai 2008 (2008-05-20), "SubName: Full=Predicted CDS Pa_3_590;" XP002510452 extrait de EBI accession no. UNIPROT:B2ACC4 Database accession no. B2ACC4
- DATABASE UniProt [Online] 15 mai 2007 (2007-05-15), "SubName: Full=Putative uncharacterized protein;" XP002510453 extrait de EBI accession no. UNIPROT:A4R3K4 Database accession no. A4R3K4
- DATABASE UniProt [Online] 21 mars 2006 (2006-03-21), "SubName: Full=Putative uncharacterized protein;" XP002510454 extrait de EBI accession no. UNIPROT:Q2GZ54 Database accession no. Q2GZ54
- BARRERA-ISLAS GASPAR A ET AL: "Characterization of a beta-glucosidase produced by a high-specific growth-rate mutant of Cellulomonas flavigena" CURRENT MICROBIOLOGY, vol. 54, no. 4, avril 2007 (2007-04), pages 266-270, XP002510449 ISSN: 0343-8651

### SEQUENCE LISTING

<110> IFP PROTEUS
<120> VARIANTS DE BETA-GLUCOSIDASE A ACTIVITE AMELIOREE ET LEURS UTILISATIONS
<130> BFF080376
<160> 18
<170> PatentIn version 3.3
<210> 1
   <211> 2235
   <212> DNA
   <213> Trichoderma reesei
<400> 1
   atgcgttacc gaacagcagc tgcgctggca cttgccactg ggccctttgc tagggcagac      60
   agtcactcaa catcgggggc ctcggctgag gcagttgtac ctcctgcagg gactccatgg     120
   ggaaccgcgt acgacaaggc gaaggccgca ttggcaaagc tcaatctcca agataaggtc     180
   ggcatcgtga gcggtgtcgg ctggaacggc ggtccttgcg ttggaaacac atctccggcc     240
   tccaagatca gctatccatc gctatgcctt caagacggac ccctcggtgt tcgatactcg     300
   acaggcagca cagcctttac gccgggcgtt caagcggcct cgacgtggga tgtcaatttg     360
   atccgcgaac gtggacagtt catcggtgag gaggtgaagg cctcggggat tcatgtcata     420
   cttggtcctg tggctgggcc gctgggaaag actccgcagg gcggtcgcaa ctgggagggc     480
   ttcggtgtcg atccatatct cacgggcatt gccatgggtc aaaccatcaa cggcatccag     540
   tcggtaggcg tgcaggcgac agcgaagcac tatatcctca acgagcagga gctcaatcga     600
   gaaaccattt cgagcaaccc agatgaccga actctccatg agctgtatac ttggccattt     660
   gccgacgcgg ttcaggccaa tgtcgcttct gtcatgtgct cgtacaacaa ggtcaatacc     720
   acctgggcct gcgaggatca gtacacgctg cagactgtgc tgaaagacca gctggggttc     780
   ccaggctatg tcatgacgga ctggaacgca cagcacacga ctgtccaaag cgcgaattct     840
   gggcttgaca tgtcaatgcc tggcacagac ttcaacggta acaatcggct ctggggtcca     900
   gctctcacca atgcggtaaa tagcaatcag gtccccacga gcagagtcga cgatatggtg     960
   actcgtatcc tcgccgcatg gtacttgaca ggccaggacc aggcaggcta tccgtcgttc    1020
   aacatcagca gaaatgttca aggaaaccac aagaccaatg tcagggcaat tgccagggac    1080
   ggcatcgttc tgctcaagaa tgacgccaac atcctgccgc tcaagaagcc cgctagcatt    1140
   gccgtcgttg gatctgccgc aatcattggt aaccacgcca gaaactcgcc ctcgtgcaac    1200
   gacaaaggct gcgacgacgg ggccttgggc atgggttggg gttccggcgc cgtcaactat    1260
   ccgtacttcg tcgcgcccta cgatgccatc aataccagag cgtcttcgca gggcacccag    1320
   gttaccttga gcaacaccga caacacgtcc tcaggcgcat ctgcagcaag aggaaaggac    1380
   gtcgccatcg tcttcatcac cgccgactcg ggtgaaggct acatcaccgt ggagggcaac    1440
   gcgggcgatc gcaacaacct ggatccgtgg cacaacggca atgccctggt ccaggcggtg   1500
   gccggtgcca acagcaacgt cattgttgtt gtccactccg ttggcgccat cattctggag   1560
   cagattcttg ctcttccgca ggtcaaggcc gttgtctggg cgggtcttcc ttctcaggag   1620
   agcggcaatg cgctcgtcga cgtgctgtgg ggagatgtca gcccttctgg caagctggtg   1680
   tacaccattg cgaagagccc caatgactat aacactcgca tcgtttccgg cggcagtgac   1740
   agcttcagcg agggactgtt catcgactat aagcacttcg acgacgccaa tatcacgccg   1800
   cggtacgagt tcggctatgg actgtcttac accaagttca actactcacg cctctccgtc   1860
   ttgtcgaccg ccaagtctgg tcctgcgact ggggccgttg tgccgggagg cccgagtgat   1920
   ctgttccaga atgtcgcgac agtcaccgtt gacatcgcaa actctggcca agtgactggt   1980
   gccgaggtag cccagctgta catcacctac ccatcttcag cacccaggac ccctccgaag   2040
   cagctgcgag gctttgccaa gctgaacctc acgcctggtc agagcggaac agcaacgttc   2100
   aacatccgac gacgagatct cagctactgg gacacggctt cgcagaaatg ggtggtgccg   2160
   tcggggtcgt ttggcatcag cgtgggagcg agcagccggg atatcaggct gacgagcact   2220
   ctgtcggtag cgtag                                                    2235
<210> 2
   <211> 744
   <212> PRT
   <213> Trichoderma reesei
<400> 2
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Val Arg Tyr Ser Thr Gly Ser Thr Ala Phe Thr Pro Gly Val Gln Ala
               100                 105                 110
   Ala Ser Thr Trp Asp Val Asn Leu Ile Arg Glu Arg Gly Gln Phe Ile
           115                 120                 125
   Gly Glu Glu Val Lys Ala Ser Gly Ile His Val Ile Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Gly Gln Thr Ile
                   165                 170                 175
   Asn Gly Ile Gln Ser Val Gly Val Gln Ala Thr Ala Lys His Tyr Ile
               180                 185                 190
   Leu Asn Glu Gln Glu Leu Asn Arg Glu Thr Ile Ser Ser Asn Pro Asp
           195                 200                 205
   Asp Arg Thr Leu His Glu Leu Tyr Thr Trp Pro Phe Ala Asp Ala Val
       210                 215                 220
   Gln Ala Asn Val Ala Ser Val Met Cys Ser Tyr Asn Lys Val Asn Thr
   225                 230                 235                 240
   Thr Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
   675 680 685
   Asn Leu Thr Pro Gly Gln Ser Gly Thr Ala Thr Phe Asn Ile Arg Arg
   690 695 700
   Arg Asp Leu Ser Tyr Trp Asp Thr Ala Ser Gln Lys Trp Val Val Pro
   705 710 715 720
   Ser Gly Ser Phe Gly Ile Ser Val Gly Ala Ser Ser Arg Asp Ile Arg
   725 730 735
   Leu Thr Ser Thr Leu Ser Val Ala
   740
<210> 3
   <211> 2235
   <212> DNA
   <213> artificial
<220>
   <223> clone 10H7
<400> 3
   atgcgttacc gaacagcagc tgcgctggca cttgccactg ggccctttgc tagggcagac 60
   agtcactcaa catcgggggc ctcggctgag gcagttgtac ctcctgcagg gactccatgg 120
   ggaaccgcgt acgacaaggc gaaggccgca ttggcaaagc tcaatctcca agataaggtc 180
   ggcatcgtga gcggtgtcgg ctggaacggc ggtccttgcg ttggaaacac atctccggcc 240
   tccaagatca gctatccatc gctatgcctt caagacggac ccctcggtat ccgattcggc 300
   acaggcagca cagcctttac gccgggcgtt caagcggcct cgacgtggga taccgagttg 360
   atgcgccagc gtggagagta cctgggtgcc gaggccaagg gctgcgggat tcatgtcctg 420
   cttggtcctg tggctggggc cctgggaaag atcccgcacg gcggtcgcaa ctgggagggc 480
   ttcggtaccg atccatatct cgccggcatt gccatggccg agacaatcga gggcctgcag 540
   tcggccggcg tgcaggcgtg cgcgaagcac tatatcgtca acgagcagga gctcaatcga 600
   gaaaccattt cgagcgacgt cgatgaccga actatgcatg agctgtatct gtggccattt 660
   gccgacgcgg ttcacgccaa tgtcgcttct gtcatgtgct cgtacaacaa ggtcaatacc 720
   acctgggcct gcgaggatca gtacacgctg cagactgtgc tgaaagacca gctggggttc 780
   ccaggctatg tcatgacgga ctggaacgca cagcacacga ctgtccaaag cgcgaattct 840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 4
   <211> 744
   <212> PRT
   <213> artificial
<220>
   <223> clone 10H7
<400> 4
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Ile Arg Phe Gly Thr Gly Ser Thr Ala Phe Thr Pro Gly Val Gln Ala
               100                 105                 110
   Ala Ser Thr Trp Asp Thr Glu Leu Met Arg Gln Arg Gly Glu Tyr Leu
           115                 120                 125
   Gly Ala Glu Ala Lys Gly Cys Gly Ile His Val Leu Leu Gly Pro Val
       130                 135                 140
   Ala Gly Ala Leu Gly Lys Ile Pro His Gly Gly Arg Asn Trp Glu Gly
   145                 150                 155                 160
   Phe Gly Thr Asp Pro Tyr Leu Ala Gly Ile Ala Met Ala Glu Thr Ile
                   165                 170                 175
   Glu Gly Leu Gln Ser Ala Gly Val Gln Ala Cys Ala Lys His Tyr Ile
               180                 185                 190
   Val Asn Glu Gln Glu Leu Asn Arg Glu Thr Ile Ser Ser Asp Val Asp
           195                 200                 205
   Asp Arg Thr Met His Glu Leu Tyr Leu Trp Pro Phe Ala Asp Ala Val
       210                 215                 220
   His Ala Asn Val Ala Ser Val Met Cys Ser Tyr Asn Lys Val Asn Thr
   225                 230                 235                 240
   Thr Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625 630 635 640
   Leu Phe Gln Asn Val Ala Thr Val Thr Val Asp Ile Ala Asn Ser Gly
   645 650 655
   Gln Val Thr Gly Ala Glu Val Ala Gln Leu Tyr Ile Thr Tyr Pro Ser
   660 665 670
   Ser Ala Pro Arg Thr Pro Pro Lys Gln Leu Arg Gly Phe Ala Lys Leu
   675 680 685
   Asn Leu Thr Pro Gly Gln Ser Gly Thr Ala Thr Phe Asn Ile Arg Arg
   690 695 700
   Arg Asp Leu Ser Tyr Trp Asp Thr Ala Ser Gln Lys Trp Val Val Pro
   705 710 715 720
   Ser Gly Ser Phe Gly Ile Ser Val Gly Ala Ser Ser Arg Asp Ile Arg
   725 730 735
   Leu Thr Ser Thr Leu Ser Val Ala
   740
<210> 5
   <211> 2232
   <212> DNA
   <213> artificial
<220>
   <223> clone 59B8
<400> 5
   atgcgttacc gaacagcagc tgcgctggca cttgccactg ggccctttgc tagggcagac     60
   agtcactcaa catcgggggc ctcggctgag gcagttgtac ctcctgcagg gactccatgg    120
   ggaaccgcgt acgacaaggc gaaggccgca ttggcaaagc tcaatctcca agataaggtc    180
   ggcatcgtga gcggtgtcgg ctggaacggc ggtccttgcg ttggaaacac atctccggcc    240
   tccaagatca gctatccatc gctatgcctt caagacggac ccctcggtat ccgattcggc    300
   acaggcagca cagcctttac gccgggcgtt caagcggcct cgacgtggga taccgagttg    360
   atgcgccagc gtggagagta cctgggtgcc gaggccaagg gctgcgggat tcatgtcctg    420
   cttggtcctg tggctggggc cctgggaaag atcccgcacg gcggtcgcaa ctgggagggc    480
   ttcggtaccg atccatatct cgccggcatt gccatggccg agacaatcga gggcctgcag    540
   tcggccggcg tgcaggcgtg cgcgaagcac tatatcgtca acgagcagga gctcaatcga    600
   gaaaccattt cgagcgacgt cgatgaccga actatgcatg agctgtatct gtggccattt    660
   gccgacgcgg ttcacgccaa tgtcgcttct gtcatgtgct cgtacaacaa ggtcaatacc    720
   acctgggcct gcgaggatca gtacacgctg cagactgtgc tgaaagacca gctggggttc    780
   ccaggctatg tcatgacgga ctggaacgca cagcacacga ctgtccaaag cgcgaattct    840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccctc aagaccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa gatcgagccg 1800
   cggtacgagt tcggcttcgg actgtcttac accgagttca cctacgccga cctctccgtc 1860
   acctcgaccg tcaccgccgg tcctgcgagc ggggagacaa tcccgggagg cgccgccgat 1920
   ctgtgggaga cagtcgcgac agtcaccgcc agcatcacca actctggcga ggtggagggt 1980
   gccgaggtag cccagctgta catcaccctg ccatctgccg cacccagcac ccctccgaag 2040
   cagctgcgag gctttgccaa gctgaagctc gagcctggtg ccagcggagt cgcaacgttc 2100
   aatctgcgac gacgagatct cagctactgg gacgccggcc gaggccagtg ggtggtgccg 2160
   gccggggagt ttaccgtcag cgtgggagcg agcagccggg atgtcaggct gacgggcagc 2220
   ctgaccgcct ag 2232
<210> 6
   <211> 743
   <212> PRT
   <213> artificial
<220>
   <223> clone 59B8
<400> 6
   Met Arg Tyr Arg Thr Ala Ala Ala Leu Ala Leu Ala Thr Gly Pro Phe
   1 5 10 15
   Ala Arg Ala Asp Ser His Ser Thr Ser Gly Ala Ser Ala Glu Ala Val
   20 25 30
   Val Pro Pro Ala Gly Thr Pro Trp Gly Thr Ala Tyr Asp Lys Ala Lys
   35 40 45
   Ala Ala Leu Ala Lys Leu Asn Leu Gln Asp Lys Val Gly Ile Val Ser
   50 55 60
   Gly Val Gly Trp Asn Gly Gly Pro Cys Val Gly Asn Thr Ser Pro Ala
   65 70 75 80
   Ser Lys Ile Ser Tyr Pro Ser Leu Cys Leu Gln Asp Gly Pro Leu Gly
   85 90 95
   Ile Arg Phe Gly Thr Gly Ser Thr Ala Phe Thr Pro Gly Val Gln Ala
   100 105 110
   Ala Ser Thr Trp Asp Thr Glu Leu Met Arg Gln Arg Gly Glu Tyr Leu
   115 120 125
   Gly Ala Glu Ala Lys Gly Cys Gly Ile His Val Leu Leu Gly Pro Val
   130 135 140
   Ala Gly Ala Leu Gly Lys Ile Pro His Gly Gly Arg Asn Trp Glu Gly
   145 150 155 160
   Phe Gly Thr Asp Pro Tyr Leu Ala Gly Ile Ala Met Ala Glu Thr Ile
   165 170 175
   Glu Gly Leu Gln Ser Ala Gly Val Gln Ala Cys Ala Lys His Tyr Ile
   180 185 190
   Val Asn Glu Gln Glu Leu Asn Arg Glu Thr Ile Ser Ser Asp Val Asp
   195 200 205
   Asp Arg Thr Met His Glu Leu Tyr Leu Trp Pro Phe Ala Asp Ala Val
   210 215 220
   His Ala Asn Val Ala Ser Val Met Cys Ser Tyr Asn Lys Val Asn Thr
   225 230 235 240
   Thr Trp Ala Cys Glu Asp Gln Tyr Thr Leu Gln Thr Val Leu Lys Asp
   245 250 255
   Gln Leu Gly Phe Pro Gly Tyr Val Met Thr Asp Trp Asn Ala Gln His
   260 265 270
   Thr Thr Val Gln Ser Ala Asn Ser Gly Leu Asp Met Ser Met Pro Gly
   275 280 285
   Thr Asp Phe Asn Gly Asn Asn Arg Leu Trp Gly Pro Ala Leu Thr Asn
   290 295 300
   Ala Val Asn Ser Asn Gln Val Pro Thr Ser Arg Val Asp Asp Met Val
   305 310 315 320
   Thr Arg Ile Leu Ala Ala Trp Tyr Leu Thr Gly Gln Asp Gln Ala Gly
   325 330 335
   Tyr Pro Ser Phe Asn Ile Ser Arg Asn Val Gln Gly Asn His Lys Thr
   340 345 350
   Asn Val Arg Ala Ile Ala Arg Asp Gly Ile Val Leu Leu Lys Asn Asp
   355 360 365
   Ala Asn Ile Leu Pro Leu Lys Lys Pro Ala Ser Ile Ala Val Val Gly
   370 375 380
   Ser Ala Ala Ile Ile Gly Asn His Ala Arg Asn Ser Pro Ser Cys Asn
   385 390 395 400
   Asp Lys Gly Cys Asp Asp Gly Ala Leu Gly Met Gly Trp Gly Ser Gly
   405 410 415
   Ala Val Asn Tyr Pro Tyr Phe Val Ala Pro Tyr Asp Ala Leu Lys Thr
   420 425 430
   Arg Ala Ser Ser Gln Gly Thr Gln Val Thr Leu Ser Asn Thr Asp Asn
   435 440 445
   Thr Ser Ser Gly Ala Ser Ala Ala Arg Gly Lys Asp Val Ala Ile Val
   450 455 460
   Phe Ile Thr Ala Asp Ser Gly Glu Gly Tyr Ile Thr Val Glu Gly Asn
   465 470 475 480
   Ala Gly Asp Arg Asn Asn Leu Asp Pro Trp His Asn Gly Asn Ala Leu
   485 490 495
   Val Gln Ala Val Ala Gly Ala Asn Ser Asn Val Ile Val Val Val His
   500 505 510
   Ser Val Gly Ala Ile Ile Leu Glu Gln Ile Leu Ala Leu Pro Gln Val
   515 520 525
   Lys Ala Val Val Trp Ala Gly Leu Pro Ser Gln Glu Ser Gly Asn Ala
   530 535 540
   Leu Val Asp Val Leu Trp Gly Asp Val Ser Pro Ser Gly Lys Leu Val
   545 550 555 560
   Tyr Thr Ile Ala Lys Ser Pro Asn Asp Tyr Asn Thr Arg Ile Val Ser
   565 570 575
   Gly Gly Ser Asp Ser Phe Ser Glu Gly Leu Phe Ile Asp Tyr Lys His
   580 585 590
   Phe Asp Asp Ala Lys Ile Glu Pro Arg Tyr Glu Phe Gly Phe Gly Leu
   595 600 605
   Ser Tyr Thr Glu Phe Thr Tyr Ala Asp Leu Ser Val Thr Ser Thr Val
   610 615 620
   Thr Ala Gly Pro Ala Ser Gly Glu Thr Ile Pro Gly Gly Ala Ala Asp
   625 630 635 640
   Leu Trp Glu Thr Val Ala Thr Val Thr Ala Ser Ile Thr Asn Ser Gly
   645 650 655
   Glu Val Glu Gly Ala Glu Val Ala Gln Leu Tyr Ile Thr Leu Pro Ser
   660 665 670
   Ala Ala Pro Ser Thr Pro Pro Lys Gln Leu Arg Gly Phe Ala Lys Leu
   675 680 685
   Lys Leu Glu Pro Gly Ala Ser Gly Val Ala Thr Phe Asn Leu Arg Arg
   690 695 700
   Arg Asp Leu Ser Tyr Trp Asp Ala Gly Arg Gly Gln Trp Val Val Pro
   705 710 715 720
   Ala Gly Glu Phe Thr Val Ser Val Gly Ala Ser Ser Arg Asp Val Arg
   725 730 735
   Leu Thr Gly Ser Leu Thr Ala
   740
<210> 7
   <211> 2235
   <212> DNA
   <213> artificial
<220>
   <223> clone 164A2
<400> 7
   atgcgttacc gaacagcagc tgcgctggca cttgccactg ggccctttgc tagggcagac 60
   agtcactcaa catcgggggc ctcggctgag gcagttgtac ctcctgcagg gactccatgg 120
   ggaaccgcgt acgacaaggc gaaggccgca ttggcaaagc tcaatctcca agataaggtc 180
   ggcatcgtga gcggtgtcgg ctggaacggc ggtccttgcg ttggaaacac atctccggcc 240
   tccaagatca gctatccatc gctatgcctt caagacggac ccctcggtgt tcgatactcg 300
   acaggcagca cagcctttac gccgggcgtt caagcggcct cgacgtggga tgtcaatttg 360
   atccgcgaac gtggacagtt catcggtgag gaggtgaagg cctcggggat tcatgtcata 420
   cttggtcctg tggctgggcc gctgggaaag actccgcagg gcggtcgcaa ctgggagggc 480
   ttcggtgtcg atccatatct cacgggcatt gccatgggtc aaaccatcaa cggcatccag 540
   tcggtaggcg tgcaggcgac agcgaagcac tatatcctca acgagcagga gctcaatcga 600
   gaaaccattt cgagcaaccc agatgaccga actctccatg agctgtatac ttggccattt 660
   gccgacgcgg ttcacgccaa tgtcgcttct gtcatgtgct cgtacaacaa gatcaatggc 720
   agctgggcct gcgaggatca gtacacgctg cagactgtgc tgaaagacca gctggggttc 780
   ccaggctatg tcatgacgga ctggaacgca cagcacacga ctgtccaaag cgcgaattct 840
   gggcttgaca tgtcaatgcc tggcacagac ttcaacggta acaatcggct ctggggtcca 900
   gctctcacca atgcggtaaa tagcaatcag gtccccacga gcagagtcga cgatatggtg 960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 8
   <211> 744
   <212> PRT
   <213> artificial
<220>
   <223> clone 164A2
<400> 8
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Val Arg Tyr Ser Thr Gly Ser Thr Ala Phe Thr Pro Gly Val Gln Ala
               100                 105                 110
   Ala Ser Thr Trp Asp Val Asn Leu Ile Arg Glu Arg Gly Gln Phe Ile
           115                 120                 125
   Gly Glu Glu Val Lys Ala Ser Gly Ile His Val Ile Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Gly Gln Thr Ile
                   165                 170                 175
   Asn Gly Ile Gln Ser Val Gly Val Gln Ala Thr Ala Lys His Tyr Ile
               180                 185                 190
   Leu Asn Glu Gln Glu Leu Asn Arg Glu Thr Ile Ser Ser Asn Pro Asp
           195                 200                 205
   Asp Arg Thr Leu His Glu Leu Tyr Thr Trp Pro Phe Ala Asp Ala Val
       210                 215                 220
   His Ala Asn Val Ala Ser Val Met Cys Ser Tyr Asn Lys Ile Asn Gly
   225                 230                 235                 240
   Ser Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
   260 265 270
   Thr Thr Val Gln Ser Ala Asn Ser Gly Leu Asp Met Ser Met Pro Gly
   275 280 285
   Thr Asp Phe Asn Gly Asn Asn Arg Leu Trp Gly Pro Ala Leu Thr Asn
   290 295 300
   Ala Val Asn Ser Asn Gln Val Pro Thr Ser Arg Val Asp Asp Met Val
   305 310 315 320
   Thr Arg Ile Leu Ala Ala Trp Tyr Leu Thr Gly Gln Asp Gln Ala Gly
   325 330 335
   Tyr Pro Ser Phe Asn Ile Ser Arg Asn Val Gln Gly Asn His Lys Thr
   340 345 350
   Asn Val Arg Ala Ile Ala Arg Asp Gly Ile Val Leu Leu Lys Asn Asp
   355 360 365
   Ala Asn Ile Leu Pro Leu Lys Lys Pro Ala Ser Ile Ala Val Val Gly
   370 375 380
   Ser Ala Ala Ile Ile Gly Asn His Ala Arg Asn Ser Pro Ser Cys Asn
   385 390 395 400
   Asp Lys Gly Cys Asp Asp Gly Ala Leu Gly Met Gly Trp Gly Ser Gly
   405 410 415
   Ala Val Asn Tyr Pro Tyr Phe Val Ala Pro Tyr Asp Ala Ile Asn Thr
   420 425 430
   Arg Ala Ser Ser Gln Gly Thr Gln Val Thr Leu Ser Asn Thr Asp Asn
   435 440 445
   Thr Ser Ser Gly Ala Ser Ala Ala Arg Gly Lys Asp Val Ala Ile Val
   450 455 460
   Phe Ile Thr Ala Asp Ser Gly Glu Gly Tyr Ile Thr Val Glu Gly Asn
   465 470 475 480
   Ala Gly Asp Arg Asn Asn Leu Asp Pro Trp His Asn Gly Asn Ala Leu
   485 490 495
   Val Gln Ala Val Ala Gly Ala Asn Ser Asn Val Ile Val Val Val His
   500 505 510
   Ser Val Gly Ala Ile Ile Leu Glu Gln Ile Leu Ala Leu Pro Gln Val
   515 520 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 9
   <211> 2235
   <212> DNA
   <213> artificial
<220>
   <223> clone 100B11
<400> 9
   atgcgttacc gaacagcagc tgcgctggca cttgccactg ggccctttgc tagggcagac 60
   agtcactcaa catcgggggc ctcggctgag gcagttgtac ctcctgcagg gactccatgg 120
   ggaaccgcgt acgacaaggc gaaggccgca ttggcaaagc tcaatctcca agataaggtc    180
   ggcatcgtga gcggtgtcgg ctggaacggc ggtccttgcg ttggaaacac atctccggcc    240
   tccaagatca gctatccatc gctatgcctt caagacggac ccctcggtat ccgattcggc    300
   acaggcagca cagcctttac gccgggcgtt caagcggcct cgacgtggga taccgagttg    360
   atgcgccagc gtggagagta cctgggtgcc gaggccaagg gctgcgggat tcatgtcctg    420
   cttggtcctg tggctgggcc gctgggaaag actccgcagg gcggtcgcaa ctgggagggc    480
   ttcggtgtcg atccatatct cacgggcatt gccatggccg agacaatcga gggcctgcag    540
   tcggccggcg tgcaggcgtg cgcgaagcac tatatcgtca acgagcagga gctcaatcga    600
   gaaaccattt cgagcgacgt cgatgaccga actatgcatg agctgtatct gtggccattt    660
   gccgacgcgg ttcaggccaa tgtcgcttct gtcatgtgct cgtacaacaa gatcaatggc    720
   agctgggcct gcgaggatca gtacacgctg cagactgtgc tgaaagacca gctggggttc    780
   ccaggctatg tcatgacgga ctggaacgca cagcacacga ctgtccaaag cgcgaattct    840
   gggcttgaca tgtcaatgcc tggcacagac ttcaacggta acaatcggct ctggggtcca    900
   gctctcacca atgcggtaaa tagcaatcag gtccccacga gcagagtcga cgatatggtg    960
   actcgtatcc tcgccgcatg gtacttgaca ggccaggacc aggcaggcta tccgtcgttc   1020
   aacatcagca gaaatgttca aggaaaccac aagaccaatg tcagggcaat tgccagggac   1080
   ggcatcgttc tgctcaagaa tgacgccaac atcctgccgc tcaagaagcc cgctagcatt   1140
   gccgtcgttg gatctgccgc aatcattggt aaccacgcca gaaactcgcc ctcgtgcaac   1200
   gacaaaggct gcgacgacgg ggccttgggc atgggttggg gttccggcgc cgtcaactat   1260
   ccgtacttcg tcgcgcccta cgatgccatc aataccagag cgtcttcgca gggcacccag   1320
   gttaccttga gcaacaccga caacacgtcc tcaggcgcat ctgcagcaag aggaaaggac   1380
   gtcgccatcg tcttcatcac cgccgactcg ggtgaaggct acatcaccgt ggagggcaac   1440
   gcgggcgatc gcaacaacct ggatccgtgg cacaacggca atgccctggt ccaggcggtg   1500
   gccggtgcca acagcaacgt cattgttgtt gtccactccg ttggcgccat cattctggag   1560
   cagattcttg ctcttccgca ggtcaaggcc gttgtctggg cgggtcttcc ttctcaggag   1620
   agcggcaatg cgctcgtcga cgtgctgtgg ggagatgtca gcccttctgg caagctggtg   1680
   tacaccattg cgaagagccc caatgactat aacactcgca tcgtttccgg cggcagtgac   1740
   agcttcagcg agggactgtt catcgactat aagcacttcg acgacgccaa tatcacgccg   1800
   cggtacgagt tcggctatgg actgtcttac accaagttca actactcacg cctctccgtc   1860
   ttttcgaccg ccaagtctgg tcctgcgact ggggccgttg tgccgggagg cccgagtgat   1920
   ctgttccaga atgtcgcgac agtcaccgtt gacatcgcaa actctggcca agtgactggt   1980
   gccgaggtag cccagctgta catcacctac ccatcttcag cacccaggac ccctccgaag   2040
   cagctgcgag gctttgccaa gctgaacctc acgcctggtc agagcggaac agcaacgttc   2100
   aacatccgac gacgagatct cagctactgg gacacggctt cgcagaaatg ggtggtgccg   2160
   tcggggtcgt ttggcatcag cgtgggagcg agcagccggg atatcaggct gacgagcact 2220
   ctgtcggtag cgtag 2235
<210> 10
   <211> 744
   <212> PRT
   <213> artificial
<220>
   <223> clone 100B11
<400> 10
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Ile Arg Phe Gly Thr Gly Ser Thr Ala Phe Thr Pro Gly Val Gln Ala
               100                 105                 110
   Ala Ser Thr Trp Asp Thr Glu Leu Met Arg Gln Arg Gly Glu Tyr Leu
           115                 120                 125
   Gly Ala Glu Ala Lys Gly Cys Gly Ile His Val Leu Leu Gly Pro Val
       130                 135                 140
   Ala Gly Pro Leu Gly Lys Thr Pro Gln Gly Gly Arg Asn Trp Glu Gly
   145                 150                 155                 160
   Phe Gly Val Asp Pro Tyr Leu Thr Gly Ile Ala Met Ala Glu Thr Ile
                   165                 170                 175
   Glu Gly Leu Gln Ser Ala Gly Val Gln Ala Cys Ala Lys His Tyr Ile
               180                 185                 190
   Val Asn Glu Gln Glu Leu Asn Arg Glu Thr Ile Ser Ser Asp Val Asp
           195                 200                 205
   Asp Arg Thr Met His Glu Leu Tyr Leu Trp Pro Phe Ala Asp Ala Val
       210                 215                 220
   Gln Ala Asn Val Ala Ser Val Met Cys Ser Tyr Asn Lys Ile Asn Gly
   225                 230                 235                 240
   Ser Trp Ala Cys Glu Asp Gln Tyr Thr Leu Gln Thr Val Leu Lys Asp
                   245                 250                 255
   Gln Leu Gly Phe Pro Gly Tyr Val Met Thr Asp Trp Asn Ala Gln His
               260                 265                 270
   Thr Thr Val Gln Ser Ala Asn Ser Gly Leu Asp Met Ser Met Pro Gly
           275                 280                 285
   Thr Asp Phe Asn Gly Asn Asn Arg Leu Trp Gly Pro Ala Leu Thr Asn
       290                 295                 300
   Ala Val Asn Ser Asn Gln Val Pro Thr Ser Arg Val Asp Asp Met Val
   305                 310                 315                 320
   Thr Arg Ile Leu Ala Ala Trp Tyr Leu Thr Gly Gln Asp Gln Ala Gly
                   325                 330                 335
   Tyr Pro Ser Phe Asn Ile Ser Arg Asn Val Gln Gly Asn His Lys Thr
               340                 345                 350
   Asn Val Arg Ala Ile Ala Arg Asp Gly Ile Val Leu Leu Lys Asn Asp
           355                 360                 365
   Ala Asn Ile Leu Pro Leu Lys Lys Pro Ala Ser Ile Ala Val Val Gly
       370                 375                 380
   Ser Ala Ala Ile Ile Gly Asn His Ala Arg Asn Ser Pro Ser Cys Asn
   385                 390                 395                 400
   Asp Lys Gly Cys Asp Asp Gly Ala Leu Gly Met Gly Trp Gly Ser Gly
                   405                 410                 415
   Ala Val Asn Tyr Pro Tyr Phe Val Ala Pro Tyr Asp Ala Ile Asn Thr
               420                 425                 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
   485 490 495
   Val Gln Ala Val Ala Gly Ala Asn Ser Asn Val Ile Val Val Val His
   500 505 510
   Ser Val Gly Ala Ile Ile Leu Glu Gln Ile Leu Ala Leu Pro Gln Val
   515 520 525
   Lys Ala Val Val Trp Ala Gly Leu Pro Ser Gln Glu Ser Gly Asn Ala
   530 535 540
   Leu Val Asp Val Leu Trp Gly Asp Val Ser Pro Ser Gly Lys Leu Val
   545 550 555 560
   Tyr Thr Ile Ala Lys Ser Pro Asn Asp Tyr Asn Thr Arg Ile Val Ser
   565 570 575
   Gly Gly Ser Asp Ser Phe Ser Glu Gly Leu Phe Ile Asp Tyr Lys His
   580 585 590
   Phe Asp Asp Ala Asn Ile Thr Pro Arg Tyr Glu Phe Gly Tyr Gly Leu
   595 600 605
   Ser Tyr Thr Lys Phe Asn Tyr Ser Arg Leu Ser Val Phe Ser Thr Ala
   610 615 620
   Lys Ser Gly Pro Ala Thr Gly Ala Val Val Pro Gly Gly Pro Ser Asp
   625 630 635 640
   Leu Phe Gln Asn Val Ala Thr Val Thr Val Asp Ile Ala Asn Ser Gly
   645 650 655
   Gln Val Thr Gly Ala Glu Val Ala Gln Leu Tyr Ile Thr Tyr Pro Ser
   660 665 670
   Ser Ala Pro Arg Thr Pro Pro Lys Gln Leu Arg Gly Phe Ala Lys Leu
   675 680 685
   Asn Leu Thr Pro Gly Gln Ser Gly Thr Ala Thr Phe Asn Ile Arg Arg
   690 695 700
   Arg Asp Leu Ser Tyr Trp Asp Thr Ala Ser Gln Lys Trp Val Val Pro
   705 710 715 720
   Ser Gly Ser Phe Gly Ile Ser Val Gly Ala Ser Ser Arg Asp Ile Arg
   725 730 735
   Leu Thr Ser Thr Leu Ser Val Ala
   740
<210> 11
   <211> 2235
   <212> DNA
   <213> artificial
<220>
   <223> clone 115E1
<400> 11
   atgcgttacc gaacagcagc tgcgctggca cttgccactg ggccctttgc tagggcagac   60
   agtcactcaa catcgggggc ctcggctgag gcagttgtac ctcctgcagg gactccatgg  120
   ggaaccgcgt acgacaaggc gaaggccgca ttggcaaagc tcaatctcca agataaggtc  180
   ggcatcgtga gcggtgtcgg ctggaacggc ggtccttgcg ttggaaacac atctccggcc  240
   tccaagatca gctatccatc gctatgcctt caagacggac ccctcggtat ccgattcggc  300
   acaggcagca cagcctttac gccgggcgtt caagcggcct cgacgtggga tgtcaatttg  360
   atccgcgaac gtggacagtt catcggtgag gaggtgaagg cctcggggat tcatgtcata  420
   cttggtcctg tggctgggcc gctgggaaag atcccgcacg gcggtcgcaa ctgggagggc  480
   ttcggtgtcg atccatatct cacgggcatt gccatggccg agacaatcga gggcctgcag  540
   tcggccggcg tgcaggcgtg cgcgaagcac tatatcctca acgagcagga gctcaatcga  600
   gaaaccattt cgagcaaccc agatgaccga actctccatg agctgtatac ttggccattt  660
   gccgacgcgg ttcacgccaa tgtcgcttct gtcatgtgct cgtacaacaa gatcaatggc  720
   agctgggcct gcgaggatca gtacacgctg cagactgtgc tgaaagacca gctggggttc  780
   ccaggctatg tcatgacgga ctggaacgca cagcacacga ctgtccaaag cgcgaattct  840
   gggcttgaca tgtcaatgcc tggcacagac ttcaacggta acaatcggct ctggggtcca  900
   gctctcacca atgcggtaaa tagcaatcag gtccccacga gcagagtcga cgatatggtg  960
   actcgtatcc tcgccgcatg gtacttgaca ggccaggacc aggcaggcta tccgtcgttc 1020
   aacatcagca gaaatgttca aggaaaccac aagaccaatg tcagggcaat tgccagggac 1080
   ggcatcgttc tgctcaagaa tgacgccaac atcctgccgc tcaagaagcc cgctagcatt 1140
   gccgtcgttg gatctgccgc aatcattggt aaccacgcca gaaactcgcc ctcgtgcaac 1200
   gacaaaggct gcgacgacgg ggccttgggc atgggttggg gttccggcgc cgtcaactat 1260
   ccgtacttcg tcgcgcccta cgatgccatc aataccagag cgtcttcgca gggcacccag 1320
   gttaccttga gcaacaccga caacacgtcc tcaggcgcat ctgcagcaag aggaaaggac 1380
   gtcgccatcg tcttcatcac cgccgactcg ggtgaaggct acatcaccgt ggagggcaac 1440
   gcgggcgatc gcaacaacct ggatccgtgg cacaacggca atgccctggt ccaggcggtg 1500
   gccggtgcca acagcaacgt cattgttgtt gtccactccg ttggcgccat cattctggag 1560
   cagattcttg ctcttccgca ggtcaaggcc gttgtctggg cgggtcttcc ttctcaggag 1620
   agcggcaatg cgctcgtcga cgtgctgtgg ggagatgtca gcccttctgg caagctggtg 1680
   tacaccattg cgaagagccc caatgactat aacactcgca tcgtttccgg cggcagtgac 1740
   agcttcagcg agggactgtt catcgactat aagcacttcg acgacgccaa tatcacgccg 1800
   cggtacgagt tcggctatgg actgtcttac accaagttca actactcacg cctctccgtc 1860
   ttgtcgaccg ccaagtctgg tcctgcgact ggggccgttg tgccgggagg cccgagtgat 1920
   ctgttccaga atgtcgcgac agtcaccgtt gacatcgcaa actctggcca agtgactggt 1980
   gccgaggtag cccagctgta catcacctac ccatcttcag cacccaggac ccctccgaag 2040
   cagctgcgag gctttgccaa gctgaacctc acgcctggtc agagcggaac agcaacgttc 2100
   aacatccgac gacgagatct cagctactgg gacacggctt cgcagaaatg ggtggtgccg 2160
   tcggggtcgt ttggcatcag cgtgggagcg agcagccggg atatcaggct gacgagcact 2220
   ctgtcggtag cgtag 2235
<210> 12
   <211> 744
   <212> PRT
   <213> artificial
<220>
   <223> clone 115E1
<400> 12
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Val
               20                  25                  30
   Val Pro Pro Ala Gly Thr Pro Trp Gly Thr Ala Tyr Asp Lys Ala Lys
           35                  40                  45
   Ala Ala Leu Ala Lys Leu Asn Leu Gln Asp Lys Val Gly Ile Val Ser
       50                  55                  60
   Gly Val Gly Trp Asn Gly Gly Pro Cys Val Gly Asn Thr Ser Pro Ala
   65                  70                  75                  80
   Ser Lys Ile Ser Tyr Pro Ser Leu Cys Leu Gln Asp Gly Pro Leu Gly
                   85                  90                  95
   Ile Arg Phe Gly Thr Gly Ser Thr Ala Phe Thr Pro Gly Val Gln Ala
               100                 105                 110
   Ala Ser Thr Trp Asp Val Asn Leu Ile Arg Glu Arg Gly Gln Phe Ile
           115                 120                 125
   Gly Glu Glu Val Lys Ala Ser Gly Ile His Val Ile Leu Gly Pro Val
       130                 135                 140
   Ala Gly Pro Leu Gly Lys Ile Pro His Gly Gly Arg Asn Trp Glu Gly
   145                 150                 155                 160
   Phe Gly Val Asp Pro Tyr Leu Thr Gly Ile Ala Met Ala Glu Thr Ile
   165 170 175
   Glu Gly Leu Gln Ser Ala Gly Val Gln Ala Cys Ala Lys His Tyr Ile
   180 185 190
   Leu Asn Glu Gln Glu Leu Asn Arg Glu Thr Ile Ser Ser Asn Pro Asp
   195 200 205
   Asp Arg Thr Leu His Glu Leu Tyr Thr Trp Pro Phe Ala Asp Ala Val
   210 215 220
   His Ala Asn Val Ala Ser Val Met Cys Ser Tyr Asn Lys Ile Asn Gly
   225 230 235 240
   Ser Trp Ala Cys Glu Asp Gln Tyr Thr Leu Gln Thr Val Leu Lys Asp
   245 250 255
   Gln Leu Gly Phe Pro Gly Tyr Val Met Thr Asp Trp Asn Ala Gln His
   260 265 270
   Thr Thr Val Gln Ser Ala Asn Ser Gly Leu Asp Met Ser Met Pro Gly
   275 280 285
   Thr Asp Phe Asn Gly Asn Asn Arg Leu Trp Gly Pro Ala Leu Thr Asn
   290 295 300
   Ala Val Asn Ser Asn Gln Val Pro Thr Ser Arg Val Asp Asp Met Val
   305 310 315 320
   Thr Arg Ile Leu Ala Ala Trp Tyr Leu Thr Gly Gln Asp Gln Ala Gly
   325 330 335
   Tyr Pro Ser Phe Asn Ile Ser Arg Asn Val Gln Gly Asn His Lys Thr
   340 345 350
   Asn Val Arg Ala Ile Ala Arg Asp Gly Ile Val Leu Leu Lys Asn Asp
   355 360 365
   Ala Asn Ile Leu Pro Leu Lys Lys Pro Ala Ser Ile Ala Val Val Gly
   370 375 380
   Ser Ala Ala Ile Ile Gly Asn His Ala Arg Asn Ser Pro Ser Cys Asn
   385 390 395 400
   Asp Lys Gly Cys Asp Asp Gly Ala Leu Gly Met Gly Trp Gly Ser Gly
   405 410 415
   Ala Val Asn Tyr Pro Tyr Phe Val Ala Pro Tyr Asp Ala Ile Asn Thr
   420 425 430
   Arg Ala Ser Ser Gln Gly Thr Gln Val Thr Leu Ser Asn Thr Asp Asn
           435                 440                 445
   Thr Ser Ser Gly Ala Ser Ala Ala Arg Gly Lys Asp Val Ala Ile Val
       450                 455                 460
   Phe Ile Thr Ala Asp Ser Gly Glu Gly Tyr Ile Thr Val Glu Gly Asn
   465                 470                 475                 480
   Ala Gly Asp Arg Asn Asn Leu Asp Pro Trp His Asn Gly Asn Ala Leu
                   485                 490                 495
   Val Gln Ala Val Ala Gly Ala Asn Ser Asn Val Ile Val Val Val His
               500                 505                 510
   Ser Val Gly Ala Ile Ile Leu Glu Gln Ile Leu Ala Leu Pro Gln Val
           515                 520                 525
   Lys Ala Val Val Trp Ala Gly Leu Pro Ser Gln Glu Ser Gly Asn Ala
       530                 535                 540
   Leu Val Asp Val Leu Trp Gly Asp Val Ser Pro Ser Gly Lys Leu Val
   545                 550                 555                 560
   Tyr Thr Ile Ala Lys Ser Pro Asn Asp Tyr Asn Thr Arg Ile Val Ser
                   565                 570                 575
   Gly Gly Ser Asp Ser Phe Ser Glu Gly Leu Phe Ile Asp Tyr Lys His
               580                 585                 590
   Phe Asp Asp Ala Asn Ile Thr Pro Arg Tyr Glu Phe Gly Tyr Gly Leu
           595                 600                 605
   Ser Tyr Thr Lys Phe Asn Tyr Ser Arg Leu Ser Val Leu Ser Thr Ala
       610                 615                 620
   Lys Ser Gly Pro Ala Thr Gly Ala Val Val Pro Gly Gly Pro Ser Asp
   625                 630                 635                 640
   Leu Phe Gln Asn Val Ala Thr Val Thr Val Asp Ile Ala Asn Ser Gly
                   645                 650                 655
   Gln Val Thr Gly Ala Glu Val Ala Gln Leu Tyr Ile Thr Tyr Pro Ser
               660                 665                 670
   Ser Ala Pro Arg Thr Pro Pro Lys Gln Leu Arg Gly Phe Ala Lys Leu
           675                 680                 685
   Asn Leu Thr Pro Gly Gln Ser Gly Thr Ala Thr Phe Asn Ile Arg Arg
       690                 695                 700
   Arg Asp Leu Ser Tyr Trp Asp Thr Ala Ser Gln Lys Trp Val Val Pro
   705                 710                 715                 720
   Ser Gly Ser Phe Gly Ile Ser Val Gly Ala Ser Ser Arg Asp Ile Arg
                   725                 730                 735
   Leu Thr Ser Thr Leu Ser Val Ala
               740
<210> 13
   <211> 2259
   <212> DNA
   <213> artificial
<220>
   <223> clone 149G7
<400> 13
   atgcgttacc gaacagcagc tgcgctggca cttgccactg ggccctttgc tagggcagac     60
   agtcactcaa catcgggggc ctcggctgag gcagagacaa gcgagaagca ggcccgacag    120
   gccggcagcg gcttcgccgc ctgggacgcc gcgtacagcc aggcgagcac cgcattgagc    180
   aagctcagcc agcaagataa ggtcaacatc gtgaccggtg tcggctggaa caagggtcct    240
   tgcgttggaa acacaccggc catcgccagc atcggctatc cacagctatg ccttcaagac    300
   ggacccctcg gtatccgatt cggcacaggc agcacagcct ttacgccggg cgttcaagcg    360
   gcctcgacgt gggatgtcga gttgatccgc cagcgtgggg tctacctggg tgccgaggcc    420
   cgaggcgtcg gggtccatgt cctgcttggt cctgtggctg ggccgctggg aaagactccg     480
   cagggcggtc gcaactggga gggcttcggt gtcgatccat atctcacggg cattgccatg    540
   agcgagacaa tcgagggcat ccagtcgaac ggcgtgcagg cgtgcgcgaa gcactatatc    600
   ctcaacgagc aggagctcaa tcgagaaacc atttcgagca acccagatga ccgaactctc    660
   catgagctgt atacttggcc atttgccgac gcggttcacg ccaatgtcgc ttctgtcatg    720
   tgctcgtaca acaagatcaa tggcagctgg gcctgcgagg atcagtacac gctgcagact    780
   gtgctgaaag accagctggg gttcccaggc tatgtcatga cggactggaa cgcacagcac    840
   acgactgtcc aaagcgcgaa ttctgggctt gacatgtcaa tgcctggcac agacttcaac    900
   ggtaacaatc ggctctgggg tccagctctc accaatgcgg taaatagcaa tcaggtcccc    960
   acgagcagag tcgacgatat ggtgactcgt atcctcgccg catggtactt gacaggccag   1020
   gaccaggcag gctatccgtc gttcaacatc agcagaaatg ttcaaggaaa ccacaagacc   1080
   aatgtcaggg caattgccag ggacggcatc gttctgctca agaatgacgc caacatcctg   1140
   ccgctcaaga agcccgctag cattgccgtc gttggatctg ccgcaatcat tggtaaccac   1200
   gccagaaact cgccctcgtg caacgacaaa ggctgcgacg acggggcctt gggcatgggt   1260
   tggggttccg gcgccgtcaa ctatccgtac ttcgtcgcgc cctacgatgc catcaatacc   1320
   agagcgtctt cgcagggcac ccaggttacc ttgagcaaca ccgacaacac gtcctcaggc   1380
   gcatctgcag caagaggaaa ggacgtcgcc atcgtcttca tcaccgccga ctcgggtgaa   1440
   ggctacatca ccgtggaggg caacgcgggc gatcgcaaca acctggatcc gtggcacaac   1500
   ggcaatgccc tggtccaggc ggtggccggt gccaacagca acgtcattgt tgttgtccac   1560
   tccgttggcg ccatcattct ggagcagatt cttgctcttc cgcaggtcaa ggccgttgtc   1620
   tgggcgggtc ttccttctca ggagagcggc aatgcgctcg tcgacgtgct gtggggagat   1680
   gtcagccctt ctggcaagct ggtgtacacc attgcgaaga gccccaatga ctataacact   1740
   cgcatcgttt ccggcggcag tgacagcttc agcgagggac tgttcatcga ctataagcac   1800
   ttcgacgacg ccaatatcac gccgcggtac gagttcggct atggactgtc ttacaccaag   1860
   ttcaactact cacgcctctc cgtcttgtcg accgccaagt ctggtcctgc gactggggcc   1920
   gttgtgccgg gaggcccgag tgatctgttc cagaatgtcg cgacagtcac cgttgacatc   1980
   gcaaactctg gccaagtgac tggtgccgag gtagcccagc tgtacatcac ctacccatct   2040
   tcagcaccca ggacccctcc gaagcagctg cgaggctttg ccaagctgaa cctcacgcct   2100
   ggtcagagcg gaacagcaac gttcaacatc cgacgacgag atctcagcta ctgggacacg   2160
   gcttcgcaga aatgggtggt gccgtcgggg tcgtttggca tcagcgtggg agcgagcagc   2220
   cgggatatca ggctgacgag cactctgtcg gtagcgtag                          2259
<210> 14
   <211> 752
   <212> PRT
   <213> artificial
<220>
   <223> clone 149G7
<400> 14
   Met Arg Tyr Arg Thr Ala Ala Ala Leu Ala Leu Ala Thr Gly Pro Phe
   1               5                   10                  15
   Ala Arg Ala Asp Ser His Ser Thr Ser Gly Ala Ser Ala Glu Ala Glu
               20                  25                  30
   Thr Ser Glu Lys Gln Ala Arg Gln Ala Gly Ser Gly Phe Ala Ala Trp
           35                  40                  45
   Asp Ala Ala Tyr Ser Gln Ala Ser Thr Ala Leu Ser Lys Leu Ser Gln
       50                  55                  60
   Gln Asp Lys Val Asn Ile Val Thr Gly Val Gly Trp Asn Lys Gly Pro
   65                  70                  75                  80
   Cys Val Gly Asn Thr Pro Ala Ile Ala Ser Ile Gly Tyr Pro Gln Leu
                   85                  90                  95
   Cys Leu Gln Asp Gly Pro Leu Gly Ile Arg Phe Gly Thr Gly Ser Thr
               100                 105                 110
   Ala Phe Thr Pro Gly Val Gln Ala Ala Ser Thr Trp Asp Val Glu Leu
           115                 120                 125
   Ile Arg Gln Arg Gly Val Tyr Leu Gly Ala Glu Ala Arg Gly Val Gly
       130                 135                 140
   Val His Val Leu Leu Gly Pro Val Ala Gly Pro Leu Gly Lys Thr Pro
   145                 150                 155                 160
   Gln Gly Gly Arg Asn Trp Glu Gly Phe Gly Val Asp Pro Tyr Leu Thr
                   165                 170                 175
   Gly Ile Ala Met Ser Glu Thr Ile Glu Gly Ile Gln Ser Asn Gly Val
               180                 185                 190
   Gln Ala Cys Ala Lys His Tyr Ile Leu Asn Glu Gln Glu Leu Asn Arg
           195                 200                 205
   Glu Thr Ile Ser Ser Asn Pro Asp Asp Arg Thr Leu His Glu Leu Tyr
       210                 215                 220
   Thr Trp Pro Phe Ala Asp Ala Val His Ala Asn Val Ala Ser Val Met
   225                 230                 235                 240
   Cys Ser Tyr Asn Lys Ile Asn Gly Ser Trp Ala Cys Glu Asp Gln Tyr
                   245                 250                 255
   Thr Leu Gln Thr Val Leu Lys Asp Gln Leu Gly Phe Pro Gly Tyr Val
               260                 265                 270
   Met Thr Asp Trp Asn Ala Gln His Thr Thr Val Gln Ser Ala Asn Ser
           275                 280                 285
   Gly Leu Asp Met Ser Met Pro Gly Thr Asp Phe Asn Gly Asn Asn Arg
       290                 295                 300
   Leu Trp Gly Pro Ala Leu Thr Asn Ala Val Asn Ser Asn Gln Val Pro
   305                 310                 315                 320
   Thr Ser Arg Val Asp Asp Met Val Thr Arg Ile Leu Ala Ala Trp Tyr
                   325                 330                 335
   Leu Thr Gly Gln Asp Gln Ala Gly Tyr Pro Ser Phe Asn Ile Ser Arg
               340                 345                 350
   Asn Val Gln Gly Asn His Lys Thr Asn Val Arg Ala Ile Ala Arg Asp
           355                 360                 365
   Gly Ile Val Leu Leu Lys Asn Asp Ala Asn Ile Leu Pro Leu Lys Lys
       370                 375                 380
   Pro Ala Ser Ile Ala Val Val Gly Ser Ala Ala Ile Ile Gly Asn His
   385 390 395 400
   Ala Arg Asn Ser Pro Ser Cys Asn Asp Lys Gly Cys Asp Asp Gly Ala
   405 410 415
   Leu Gly Met Gly Trp Gly Ser Gly Ala Val Asn Tyr Pro Tyr Phe Val
   420 425 430
   Ala Pro Tyr Asp Ala Ile Asn Thr Arg Ala Ser Ser Gln Gly Thr Gln
   435 440 445
   Val Thr Leu Ser Asn Thr Asp Asn Thr Ser Ser Gly Ala Ser Ala Ala
   450 455 460
   Arg Gly Lys Asp Val Ala Ile Val Phe Ile Thr Ala Asp Ser Gly Glu
   465 470 475 480
   Gly Tyr Ile Thr Val Glu Gly Asn Ala Gly Asp Arg Asn Asn Leu Asp
   485 490 495
   Pro Trp His Asn Gly Asn Ala Leu Val Gln Ala Val Ala Gly Ala Asn
   500 505 510
   Ser Asn Val Ile Val Val Val His Ser Val Gly Ala Ile Ile Leu Glu
   515 520 525
   Gln Ile Leu Ala Leu Pro Gln Val Lys Ala Val Val Trp Ala Gly Leu
   530 535 540
   Pro Ser Gln Glu Ser Gly Asn Ala Leu Val Asp Val Leu Trp Gly Asp
   545 550 555 560
   Val Ser Pro Ser Gly Lys Leu Val Tyr Thr Ile Ala Lys Ser Pro Asn
   565 570 575
   Asp Tyr Asn Thr Arg Ile Val Ser Gly Gly Ser Asp Ser Phe Ser Glu
   580 585 590
   Gly Leu Phe Ile Asp Tyr Lys His Phe Asp Asp Ala Asn Ile Thr Pro
   595 600 605
   Arg Tyr Glu Phe Gly Tyr Gly Leu Ser Tyr Thr Lys Phe Asn Tyr Ser
   610 615 620
   Arg Leu Ser Val Leu Ser Thr Ala Lys Ser Gly Pro Ala Thr Gly Ala
   625 630 635 640
   Val Val Pro Gly Gly Pro Ser Asp Leu Phe Gln Asn Val Ala Thr Val
   645 650 655
   Thr Val Asp Ile Ala Asn Ser Gly Gln Val Thr Gly Ala Glu Val Ala
               660                 665                 670
   Gln Leu Tyr Ile Thr Tyr Pro Ser Ser Ala Pro Arg Thr Pro Pro Lys
           675                 680                 685
   Gln Leu Arg Gly Phe Ala Lys Leu Asn Leu Thr Pro Gly Gln Ser Gly
       690                 695                 700
   Thr Ala Thr Phe Asn Ile Arg Arg Arg Asp Leu Ser Tyr Trp Asp Thr
   705                 710                 715                 720
   Ala Ser Gln Lys Trp Val Val Pro Ser Gly Ser Phe Gly Ile Ser Val
                   725                 730                 735
   Gly Ala Ser Ser Arg Asp Ile Arg Leu Thr Ser Thr Leu Ser Val Ala
               740                 745                 750
<210> 15
   <211> 2181
   <212> DNA
   <213> Chaetomium globosum
<400> 15
   atgacgacgc tccgcaactt tgcgctgctc gcagcggcgg tgcttgcgcg ggtcgaggcc 60
   ctcgaggccg ccgactgggc tgcggctgag gcctcagcca aaaccgcact ggcaaagatg 120
   tcacaacaag acaaaatcag cattgtgacg ggcatcggct gggacaaggg tccctgtgtc 180
   ggcaacacgg ccgccatcaa ctcgatcaac tacccgcagc tctgcctaca ggacggcccg 240
   ctcgggatcc gcttcggcac cggctcgacg gccttcaccc cgggcgtcca agccgcctcg 300
   acatgggata ccgagctgat gcgccagcgc ggcgagtacc tcggggccga ggccaagggg 360
   tgcggcatcc acgtgttgct gggccccgtg gccggggcac tgggcaagat cccgcacggc 420
   gggcgcaact gggaaggatt cgggacggac ccgtacctgg cgggcatcgc catggccgag 480
   acgatcgagg ggctgcagtc ggcgggggtg caggcgtgcg ccaagcacta catcgtcaac 540
   gagcaggagc tcaaccgcga gaccatcagc agcgacgtcg acgaccgcac catgcacgag 600
   ctgtacctgt ggcccttcgc cgacgccgtg cacgccaacg tggccagcgt catgtgcagc 660
   tacaacaaga tcaacggctc gtggggctgc gagaacgacc acgcccaaaa cggcctgctc 720
   aagaaggagc tcggcttcaa gggttacgtc gtcagcgact ggaacgcgca gcacacgacc 780
   gacggcgccg ccaacaacgg catggacatg accatgccgg gcagcgacta caacggcaac 840
   aacgtgctct ggggcccgca gctcagcaac gccgtcaaca gcaaccgggt ctcgcgcgac 900
   cggctcgacg acatggccaa acgcatcctc acctcatggt acctcctggg ccagaactcg 960
   ggctacccca acatcaacat caacgccaac gtgcagggca accacaagga gaacgtgcgg 1020
   gcggtggcgc gcgacggcat cgtgctgctc aagaacgacg agggcgtgct cccgctgaag 1080
   aagccaggca aggtggctct cgtcggatcg gcggcctcgg tcaacagcgc gggccccaac 1140
   gcgtgcgtcg acaagggctg caacacgggc gcgctcggca tgggctgggg gtccgggtcc 1200
   gtcaactacc cctactttgt ggcgccctac gacgcgctca agacgcgcgc ccaggccgac 1260
   ggcaccacgc tcagcctgca caactcggac tcgaccaacg gcgtatcggg cgtggtgtcg 1320
   ggcgccgacg tggccatcgt ggtgatcacg gcggactcgg gcgagggcta catcacggtc 1380
   gagggccacg ccggcgaccg caaccacctg gacccgtggc acgacggcaa cgcgctggtt 1440
   aaggcggtgg ccgcggccaa caagaacacc atcgtggtag tgcacagcac agggcccatc 1500
   atcctcgaga ccatcctggc gacggagggt gtcaaggcgg ttgtgtgggc cggcctgccg 1560
   agtcaggaga acggcaacgc gctagttgac gttttgtacg gcctgacttc gccctcaggc 1620
   aaactggtct actccatcgc caagcgcccc gaggactatg gcacggcccc ctccaagggc 1680
   agtaacgaca agttcaccga aggcctgttt gtcgactacc ggcactttga caacgccaag 1740
   attgagccgc ggtacgagtt tggctttggt ttgtcctaca ccgaattcac ctacgccgac 1800
   ctctccgtca cttccaccgt aacggccggc cccgcctcag gcgagaccat acccggcggc 1860
   gcggccgacc tctgggagac tgtcgcaacg gtcacggcgt ccatcacgaa cagcggcgag 1920
   gtggagggcg ccgaggtggc gcagctgtac atcacgctgc cgtcggcggc cccctcgacg 1980
   ccgcccaagc agctgcgcgg gttcgccaag ctcaagctcg agccgggggc gtcgggcgtc 2040
   gcgaccttca acctgcgccg tcgcgatctg agttattggg atgccgggcg cggccagtgg 2100
   gtggtgccgg cgggcgagtt tacggtttcg gttggtgcga gttcgaggga tgtgcgcttg 2160
   acggggagct tgactgctta g 2181
<210> 16
   <211> 726
   <212> PRT
   <213> Chaetomium globosum
<400> 16
   Met Thr Thr Leu Arg Asn Phe Ala Leu Leu Ala Ala Ala Val Leu Ala
   1               5                   10                  15
   Arg Val Glu Ala Leu Glu Ala Ala Asp Trp Ala Ala Ala Glu Ala Ser
               20                  25                  30
   Ala Lys Thr Ala Leu Ala Lys Met Ser Gln Gln Asp Lys Ile Ser Ile
           35                  40                  45
   Val Thr Gly Ile Gly Trp Asp Lys Gly Pro Cys Val Gly Asn Thr Ala
       50                  55                  60
   Ala Ile Asn Ser Ile Asn Tyr Pro Gln Leu Cys Leu Gln Asp Gly Pro
   65                  70                  75                  80
   Leu Gly Ile Arg Phe Gly Thr Gly Ser Thr Ala Phe Thr Pro Gly Val
                   85                  90                  95
   Gln Ala Ala Ser Thr Trp Asp Thr Glu Leu Met Arg Gln Arg Gly Glu
               100                 105                 110
   Tyr Leu Gly Ala Glu Ala Lys Gly Cys Gly Ile His Val Leu Leu Gly
           115                 120                 125
   Pro Val Ala Gly Ala Leu Gly Lys Ile Pro His Gly Gly Arg Asn Trp
       130                 135                 140
   Glu Gly Phe Gly Thr Asp Pro Tyr Leu Ala Gly Ile Ala Met Ala Glu
   145                 150                 155                 160
   Thr Ile Glu Gly Leu Gln Ser Ala Gly Val Gln Ala Cys Ala Lys His
                   165                 170                 175
   Tyr Ile Val Asn Glu Gln Glu Leu Asn Arg Glu Thr Ile Ser Ser Asp
               180                 185                 190
   Val Asp Asp Arg Thr Met His Glu Leu Tyr Leu Trp Pro Phe Ala Asp
           195                 200                 205
   Ala Val His Ala Asn Val Ala Ser Val Met Cys Ser Tyr Asn Lys Ile
       210                 215                 220
   Asn Gly Ser Trp Gly Cys Glu Asn Asp His Ala Gln Asn Gly Leu Leu
   225                 230                 235                 240
   Lys Lys Glu Leu Gly Phe Lys Gly Tyr Val Val Ser Asp Trp Asn Ala
                   245                 250                 255
   Gln His Thr Thr Asp Gly Ala Ala Asn Asn Gly Met Asp Met Thr Met
               260                 265                 270
   Pro Gly Ser Asp Tyr Asn Gly Asn Asn Val Leu Trp Gly Pro Gln Leu
           275                 280                 285
   Ser Asn Ala Val Asn Ser Asn Arg Val Ser Arg Asp Arg Leu Asp Asp
       290                 295                 300
   Met Ala Lys Arg Ile Leu Thr Ser Trp Tyr Leu Leu Gly Gln Asn Ser
   305                 310                 315                 320
   Gly Tyr Pro Asn Ile Asn Ile Asn Ala Asn Val Gln Gly Asn His Lys
                   325                 330                 335
   Glu Asn Val Arg Ala Val Ala Arg Asp Gly Ile Val Leu Leu Lys Asn
               340                 345                 350
   Asp Glu Gly Val Leu Pro Leu Lys Lys Pro Gly Lys Val Ala Leu Val
           355                 360                 365
   Gly Ser Ala Ala Ser Val Asn Ser Ala Gly Pro Asn Ala Cys Val Asp
       370                 375                 380
   Lys Gly Cys Asn Thr Gly Ala Leu Gly Met Gly Trp Gly Ser Gly Ser
   385                 390                 395                 400
   Val Asn Tyr Pro Tyr Phe Val Ala Pro Tyr Asp Ala Leu Lys Thr Arg
                   405                 410                 415
   Ala Gln Ala Asp Gly Thr Thr Leu Ser Leu His Asn Ser Asp Ser Thr
               420                 425                 430
   Asn Gly Val Ser Gly Val Val Ser Gly Ala Asp Val Ala Ile Val Val
           435                 440                 445
   Ile Thr Ala Asp Ser Gly Glu Gly Tyr Ile Thr Val Glu Gly His Ala
       450                 455                 460
   Gly Asp Arg Asn His Leu Asp Pro Trp His Asp Gly Asn Ala Leu Val
   465                 470                 475                 480
   Lys Ala Val Ala Ala Ala Asn Lys Asn Thr Ile Val Val Val His Ser
                   485                 490                 495
   Thr Gly Pro Ile Ile Leu Glu Thr Ile Leu Ala Thr Glu Gly Val Lys
               500                 505                 510
   Ala Val Val Trp Ala Gly Leu Pro Ser Gln Glu Asn Gly Asn Ala Leu
           515                 520                 525
   Val Asp Val Leu Tyr Gly Leu Thr Ser Pro Ser Gly Lys Leu Val Tyr
       530                 535                 540
   Ser Ile Ala Lys Arg Pro Glu Asp Tyr Gly Thr Ala Pro Ser Lys Gly
   545                 550                 555                 560
   Ser Asn Asp Lys Phe Thr Glu Gly Leu Phe Val Asp Tyr Arg His Phe
                   565                 570                 575
   Asp Asn Ala Lys Ile Glu Pro Arg Tyr Glu Phe Gly Phe Gly Leu Ser
               580                 585                 590
   Tyr Thr Glu Phe Thr Tyr Ala Asp Leu Ser Val Thr Ser Thr Val Thr
           595                 600                 605
   Ala Gly Pro Ala Ser Gly Glu Thr Ile Pro Gly Gly Ala Ala Asp Leu
       610                 615                 620
   Trp Glu Thr Val Ala Thr Val Thr Ala Ser Ile Thr Asn Ser Gly Glu
   625                 630                 635                 640
   Val Glu Gly Ala Glu Val Ala Gln Leu Tyr Ile Thr Leu Pro Ser Ala
   645 650 655
   Ala Pro Ser Thr Pro Pro Lys Gln Leu Arg Gly Phe Ala Lys Leu Lys
   660 665 670
   Leu Glu Pro Gly Ala Ser Gly Val Ala Thr Phe Asn Leu Arg Arg Arg
   675 680 685
   Asp Leu Ser Tyr Trp Asp Ala Gly Arg Gly Gln Trp Val Val Pro Ala
   690 695 700
   Gly Glu Phe Thr Val Ser Val Gly Ala Ser Ser Arg Asp Val Arg Leu
   705 710 715 720
   Thr Gly Ser Leu Thr Ala
   725
<210> 17
   <211> 2208
   <212> DNA
   <213> Neurospora crassa
<400> 17
   atgcaccttc gaatatttgc ggtgttggcc gcgacttccc tcgcctgggc cgagactagc 60
   gagaaacaag ctcgtcaagc tggctcaggt tttgcggcgt gggacgcagc ctattctcag 120
   gcaagcactg ctctctccaa gctttcacag caagacaagg tcaacatcgt caccggagtc 180
   ggctggaata agggcccatg tgttggcaac accccagcta ttgcatcaat cggttatccc 240
   cagctctgtt tacaagacgg ccctctcggc attcggtttg gaggaagtgt caccgcgttc 300
   acgcctggta tccaggcggc ttcaacatgg gacgtcgaac tgattcgaca gcgcggcgtc 360
   tacctcggtg cagaagccag aggggttggc gtacatgtcc ttcttggacc cgtggccgga 420
   gcgcttggca agatccccaa tggtggacgt aactgggagg gctttggtcc ggatccctac 480
   ctcacaggta ttgccatgag cgaaacaatt gaagggatcc agagcaatgg tgtacaagct 540
   tgcgccaagc acttcattct caacgaacag gagacaaacc gcgatactat cagcagtgtc 600
   gtcgacgacc gcaccatgca tgaactatac ctcttccctt ttgccgatgc cgtacactca 660
   aatgttgcaa gtgtgatgtg cagctacaac aaggtcaacg gtacgtgggc atgtgagaat 720
   gacaaaatcc agaatggcct tctcaagaaa gagctaggct tcaaaggata tgtcatgagt 780
   gattggaacg cccagcacac cacgaacggc gctgcaaaca gtggtatgga tatgacgatg 840
   ccaggcagtg actttaatgg caagacgatc ctgtggggac cacagctcaa caccgccgtc 900
   aacaatggcc aggtctccaa agcaagactg gacgacatgg ccaagcgcat tctcgcatcg 960
   tggtatttac tcgagcaaaa ctcaggctac cctgcgacta acctcaaggc caatgttcaa 1020
   ggaaaccaca aggagaacgt tcgcgcagtg gcaagagacg gcattgttct gctgaagaac 1080
   gacgataaca tcctcccgct caagaagcct agcaagctgg caatcattgg gtcatcgtcc 1140
   gttgtcaacc ctgcgggaag gaacgcctgc accgatcgag gatgcaacac cggtgcgctc    1200
   ggcatgggtt ggggctccgg cacggccgat tacccctact tcgtagcacc ctatgatgct    1260
   ctcaagacgc gggctcagtc cgacggaaca actgtcaacc tactcagctc tgacagcacc    1320
   agcggcgtag ccaacgctgc ctccggagcc gacgcggcac tagtcttcat cacagccgat    1380
   tccggcgaag gctacatcac ggtcgagggc gtgaccggcg accgtcccaa cctcgatccc    1440
   tggcacaacg gcaaccagct agtccaagcc gtggctcaag ccaacaagaa caccattgtc    1500
   gtcgtccaca gtaccggccc catcattctg gagactatcc tcgcgcagcc gggcgtcaag    1560
   gcggtcgtgt gggccggtct ccccagccaa gagaacggca acgcccttgt cgatgtccta    1620
   tacggcttgg tctctccctc gggtaagctg ccgtatacta tcgccaagag cgaaagcgac    1680
   tacggcactg ccgtgcaaag gggagggacg gatctgttca ctgagggtct gttcatcgat    1740
   taccgccact ttgacaagaa cggtatcgct ccccggtatg agttcggttt cggtctttcc    1800
   tacacgaact tcacctactc ctccctctcc atcacctcca ccgcctcctc cggtcccgcc    1860
   tcgggtgaca ccatccctgg cggccgcgcc gacctctggg aaaccgtggc aaccgtcact    1920
   gccgtcgtca aaaacacggg tggtgtgcag ggcgccgagg caccccagct atacatcacc    1980
   ttgccctctt ccgcgccgtc gagcccgccg aaacagctca gagggtttgc aaagctgaag    2040
   ctggcgcccg gggagagcaa gacagctacg ttcattttgc ggaggaggga tttgagttat    2100
   tgggatacgg gcagccagaa ttgggtggtg cctagtggca gctttggggt ggtagtgggt    2160
   gctagttcga gggatttgag gttgaatggg aagtttgatg tttattga                2208
<210> 18
   <211> 735
   <212> PRT
   <213> Neurospora crassa
<400> 18
   Met His Leu Arg Ile Phe Ala Val Leu Ala Ala Thr Ser Leu Ala Trp
   1               5                   10                  15
   Ala Glu Thr Ser Glu Lys Gln Ala Arg Gln Ala Gly Ser Gly Phe Ala
               20                  25                  30
   Ala Trp Asp Ala Ala Tyr Ser Gln Ala Ser Thr Ala Leu Ser Lys Leu
           35                  40                  45
   Ser Gln Gln Asp Lys Val Asn Ile Val Thr Gly Val Gly Trp Asn Lys
       50                  55                  60
   Gly Pro Cys Val Gly Asn Thr Pro Ala Ile Ala Ser Ile Gly Tyr Pro
   65                  70                  75                  80
   Gln Leu Cys Leu Gln Asp Gly Pro Leu Gly Ile Arg Phe Gly Gly Ser
                   85                  90                  95
   Val Thr Ala Phe Thr Pro Gly Ile Gln Ala Ala Ser Thr Trp Asp Val
               100                 105                 110
   Glu Leu Ile Arg Gln Arg Gly Val Tyr Leu Gly Ala Glu Ala Arg Gly
           115                 120                 125
   Val Gly Val His Val Leu Leu Gly Pro Val Ala Gly Ala Leu Gly Lys
       130                 135                 140
   Ile Pro Asn Gly Gly Arg Asn Trp Glu Gly Phe Gly Pro Asp Pro Tyr
   145                 150                 155                 160
   Leu Thr Gly Ile Ala Met Ser Glu Thr Ile Glu Gly Ile Gln Ser Asn
                   165                 170                 175
   Gly Val Gln Ala Cys Ala Lys His Phe Ile Leu Asn Glu Gln Glu Thr
               180                 185                 190
   Asn Arg Asp Thr Ile Ser Ser Val Val Asp Asp Arg Thr Met His Glu
           195                 200                 205
   Leu Tyr Leu Phe Pro Phe Ala Asp Ala Val His Ser Asn Val Ala Ser
       210                 215                 220
   Val Met Cys Ser Tyr Asn Lys Val Asn Gly Thr Trp Ala Cys Glu Asn
   225                 230                 235                 240
   Asp Lys Ile Gln Asn Gly Leu Leu Lys Lys Glu Leu Gly Phe Lys Gly
                   245                 250                 255
   Tyr Val Met Ser Asp Trp Asn Ala Gln His Thr Thr Asn Gly Ala Ala
               260                 265                 270
   Asn Ser Gly Met Asp Met Thr Met Pro Gly Ser Asp Phe Asn Gly Lys
           275                 280                 285
   Thr Ile Leu Trp Gly Pro Gln Leu Asn Thr Ala Val Asn Asn Gly Gln
       290                 295                 300
   Val Ser Lys Ala Arg Leu Asp Asp Met Ala Lys Arg Ile Leu Ala Ser
   305                 310                 315                 320
   Trp Tyr Leu Leu Glu Gln Asn Ser Gly Tyr Pro Ala Thr Asn Leu Lys
                   325                 330                 335
   Ala Asn Val Gln Gly Asn His Lys Glu Asn Val Arg Ala Val Ala Arg
               340                 345                 350
   Asp Gly Ile Val Leu Leu Lys Asn Asp Asp Asn Ile Leu Pro Leu Lys
           355                 360                 365
   Lys Pro Ser Lys Leu Ala Ile Ile Gly Ser Ser Ser Val Val Asn Pro
   370 375 380
   Ala Gly Arg Asn Ala Cys Thr Asp Arg Gly Cys Asn Thr Gly Ala Leu
   385 390 395 400
   Gly Met Gly Trp Gly Ser Gly Thr Ala Asp Tyr Pro Tyr Phe Val Ala
   405 410 415
   Pro Tyr Asp Ala Leu Lys Thr Arg Ala Gln Ser Asp Gly Thr Thr Val
   420 425 430
   Asn Leu Leu Ser Ser Asp Ser Thr Ser Gly Val Ala Asn Ala Ala Ser
   435 440 445
   Gly Ala Asp Ala Ala Leu Val Phe Ile Thr Ala Asp Ser Gly Glu Gly
   450 455 460
   Tyr Ile Thr Val Glu Gly Val Thr Gly Asp Arg Pro Asn Leu Asp Pro
   465 470 475 480
   Trp His Asn Gly Asn Gln Leu Val Gln Ala Val Ala Gln Ala Asn Lys
   485 490 495
   Asn Thr Ile Val Val Val His Ser Thr Gly Pro Ile Ile Leu Glu Thr
   500 505 510
   Ile Leu Ala Gln Pro Gly Val Lys Ala Val Val Trp Ala Gly Leu Pro
   515 520 525
   Ser Gln Glu Asn Gly Asn Ala Leu Val Asp Val Leu Tyr Gly Leu Val
   530 535 540
   Ser Pro Ser Gly Lys Leu Pro Tyr Thr Ile Ala Lys Ser Glu Ser Asp
   545 550 555 560
   Tyr Gly Thr Ala Val Gln Arg Gly Gly Thr Asp Leu Phe Thr Glu Gly
   565 570 575
   Leu Phe Ile Asp Tyr Arg His Phe Asp Lys Asn Gly Ile Ala Pro Arg
   580 585 590
   Tyr Glu Phe Gly Phe Gly Leu Ser Tyr Thr Asn Phe Thr Tyr Ser Ser
   595 600 605
   Leu Ser Ile Thr Ser Thr Ala Ser Ser Gly Pro Ala Ser Gly Asp Thr
   610 615 620
   Ile Pro Gly Gly Arg Ala Asp Leu Trp Glu Thr Val Ala Thr Val Thr
   625 630 635 640
   Ala Val Val Lys Asn Thr Gly Gly Val Gln Gly Ala Glu Ala Pro Gln
                   645                 650                 655
   Leu Tyr Ile Thr Leu Pro Ser Ser Ala Pro Ser Ser Pro Pro Lys Gln
               660                 665                 670
   Leu Arg Gly Phe Ala Lys Leu Lys Leu Ala Pro Gly Glu Ser Lys Thr
           675                 680                 685
   Ala Thr Phe Ile Leu Arg Arg Arg Asp Leu Ser Tyr Trp Asp Thr Gly
       690                 695                 700
   Ser Gln Asn Trp Val Val Pro Ser Gly Ser Phe Gly Val Val Val Gly
   705                 710                 715                 720
   Ala Ser Ser Arg Asp Leu Arg Leu Asn Gly Lys Phe Asp Val Tyr
                   725                 730                 735

## Description

La présente invention porte sur l'expression et l'optimisation d'enzymes impliquées dans la dégradation de la biomasse lignocellulosique. La présente invention porte plus particulièrement sur des variants de la beta-glucosidase comportant au moins une altération parmi les acides aminés situés en position 225, 238, 240 et 241 selon la numérotation de la SEQ ID :2 de la beta-glucosidase de *Trichoderma reesei,* ainsi que sur l'utilisation de ces variants à performance améliorée dans des procédés de dégradation de la cellulose et des procédés de production de biocarburant (par exemple : éthanol, butanol, isopropanol).

La possibilité de produire de l'éthanol à partir de la cellulose a reçu beaucoup d'attention en raison de la disponibilité de grandes quantités de matière première ainsi que de la propreté de l'éthanol à titre de carburant.

Les matières premières naturelles cellulosiques pour un tel processus sont désignées par le terme "biomasse". De nombreux types de biomasse, y compris le bois, les résidus agricoles, les cultures herbacées et les déchets solides municipaux, ont été considérés comme des matières premières pour la production de biocarburant. Ces matériaux sont constitués principalement de cellulose, d'hémicellulose et de lignine.

La cellulose, est un polymère constitué de molécules de glucose reliées par des liens beta 1-4, qui est très résistant à la dégradation ou à la dépolymérisation à l'aide d'acides, d'enzymes, ou de microorganismes. Une fois la cellulose convertie en glucose, celui-ci est facilement fermenté en biocarburant, par exemple l'éthanol, en utilisant une levure.

Les plus anciennes méthodes étudiées pour convertir la cellulose en glucose sont basées sur l'hydrolyse acide. Ce processus peut se faire en présence de concentrés d'acides ou d'acides dilués. Cependant, plusieurs inconvénients tels que la mauvaise récupération de l'acide lors de l'utilisation des concentrés d'acides et la faible production de glucose dans le cadre de l'utilisation d'acides dilués empêchent le processus d'hydrolyse acide de parvenir à la commercialisation.

Pour surmonter les inconvénients du processus d'hydrolyse acide, les processus de conversion de la cellulose ont porté plus récemment sur l'hydrolyse enzymatique, à l'aide d'enzymes de type cellulase. Cette hydrolyse enzymatique de la biomasse lignocellulosique (par exemple, la cellulose) présente cependant l'inconvénient d'être un procédé industriel coûteux. De ce fait, il est nécessaire d'utiliser des souches de microorganismes sécréteurs de cellulases de plus en plus performantes.

A ce titre, beaucoup de microorganismes comportent des enzymes qui hydrolysent la cellulose, tels que les champignons *Trichoderma, Aspergillus, Humicola, Fusarium* ainsi que les bactéries telles que *Thermomonospora, Bacillus, Cellulomonas* et *Streptomyces.* Les enzymes présentes dans ces microorganismes possèdent trois types d'activités utiles dans la conversion de la cellulose en glucose et se divisent en trois groupes: les endoglucanases, qui attaquent les fibres de celluloses aléatoirement en interne, les exoglucanases qui vont attaquer les extrémités des fibres en libérant du cellobiose, et les beta-glucosidases qui vont hydrolyser ce cellobiose en glucose. Ces dernières constituent l'étape limitante du procédé de conversion de la cellulose. En effet, la difficulté première du procédé réside dans la conversion du cellobiose en glucose, car tout cellobiose non hydrolysé à la fin du procédé représente une perte de rendement lors de la production de biocarburant.

Cette accumulation de cellobiose est un problème majeur dans l'hydrolyse enzymatique, étant donné que plusieurs microorganismes producteurs de cellulases, dont *Trichoderma,* produisent très peu de beta-glucosidase. En fait, moins de 1% des protéines totales produites par des souches de *Trichoderma* industrielles sont de type beta-glucosidase. Cette faible quantité de beta-glucosidase résulte donc en une faible capacité à hydrolyser le cellobiose en glucose ; de là, son accumulation dans le système. Or une forte concentration de cellobiose inhibe l'activité des autres cellulases et notamment les exoglucanases pour qui le cellobiose est le produit final de réaction.

Plusieurs approches ont été proposées pour augmenter l'activité beta-glucosidase dans les microorganismes et par conséquent la conversion du cellobiose en glucose.

Une première approche consiste à ajouter aux mélanges sécrétés par les microorganismes de la beta-glucosidase produite de façon exogène, pour améliorer l'hydrolyse. Cependant, ce procédé n'est pas viable commercialement, car beaucoup trop coûteux.

Une seconde approche, telle que décrite dans WO92/010581, est d'utiliser le génie génétique pour insérer de nouvelles copies du gène de la beta-glucosidase dans le génome des microorganismes, de façon à ce que ces derniers produisent une plus grande quantité d'enzyme.

Une troisième approche, décrite dans WO99/46362, consiste à modifier génétiquement les microorganismes à l'aide d'une construction génétique qui comprend un promoteur, le gène de la beta-glucosidase mature et la séquence signal de sécrétion de la xylanase. La présence de la séquence signal de sécrétion de la xylanase permet d'augmenter de manière significative la quantité de beta-glucosidase produite par les microorganismes.

Or, pour qu'une hydrolyse de la biomasse lignocellulosique soit efficace et économiquement rentable, le mélange enzymatique doit être produit par une seule et unique souche microbienne, et doit comporter des proportions équilibrées d'activités enzymatiques diverses (entre autres, mais non exclusivement exoglucanases, endoglucanases, xylanases et beta-glucosidases). A titre d'exemple, dans les mélanges natifs de *Trichoderma reesei* on constate généralement la présence de 70-80% d'exoglucanases, 15-20% d'endoglucanases, quelques pourcentages d'hémicellulases et environ 0,5% de beta-glucosidases. Ce mélange convient parfaitement pour hydrolyser la majorité des substrats prétraités (ex. type paille de blé explosée à la vapeur en conditions acides) avec des rendements acceptables. En somme, l'augmentation de l'activité beta-glucosidase, si elle est faite par enrichissement en quantité d'enzyme, ne doit pas se faire au détriment des autres activités enzymatiques.

En conséquence, la possibilité d'obtenir de hautes activités beta-glucosidase sans modifier notablement la proportion de l'ensemble des enzymes du mélange serait un gain significatif pour le procédé de conversion de biomasse lignocellulosique en biocarburant.

L'art antérieur fait état de plusieurs séquences codant pour des beta glucosidases modifiées. Ces séquences sont disponibles sous les numéros d'accession EBI : ADM94194, B2ACC4, A4R3K4 et Q2GZ54.

Les sociétés déposantes, ont eu le grand mérite de trouver, après de nombreuses recherches, un polypeptide isolé ou purifié ayant une activité beta-glucosidase améliorée par rapport à l'activité beta-glucosidase de la protéine sauvage BGL1 (SEQ ID NO :2), comprenant une séquence d'acides aminés dans laquelle au moins un acide aminé est altéré par rapport à la séquence d'acides aminés selon SEQ ID No :2, ledit acide aminé altéré étant choisi parmi les positions 225, 238, 240 et 241 de la séquence d'acides aminés SEQ ID No :2, et la dite séquence d'acides aminés ayant au moins 75% d'identité de séquence avec SEQ ID No :2 et ledit polypeptide étant choisi dans le groupe constitué de
- une séquence d'acides aminés choisie parmi SEQ ID No :4, SEQ ID NO :6, SEQ ID NO :8, SEQ ID NO :10, SEQ ID NO :12; SEQ ID NO :14;
- une séquence d'acides aminés ayant au moins 90% d'identité, préférentiellement 95%, 98% ou 99% avec la séquence SEQ ID N°2 et comprenant l'altération Q225H;
- une séquence d'acides aminés ayant au moins 90% d'identité, préférentiellement 95%, 98% ou 99% avec la séquence SEQ ID N°2 et comprenant les altérations V238I, T240G et T241S; et
- une séquence d'acides aminés ayant au moins 90% d'identité, préférentiellement 95%, 98% ou 99% avec la séquence SEQ ID N°2 et comprenant les altérations Q225H, V238I, T240G et T241S.

De plus, les polypeptides selon l'invention présentent l'avantage d'être moins sensibles à l'inhibition par le glucose et conservent de ce fait une meilleure activité beta-glucosidase en présence d'une forte concentration de glucose.

Dans un mode de réalisation, le polypeptide tel que décrit précédemment est caractérisé en ce qu'il a une activité beta-glucosidase déterminée en présence de glucose qui est améliorée par rapport à l'activité beta-glucosidase de la protéine sauvage BGL1 (SEQ ID NO :2) déterminée en absence de glucose.

L'homme du métier pourra par exemple déterminer l'augmentation ou autrement dit l'amélioration de l'activité enzymatique d'un polypeptide selon l'invention par un test d'activité enzymatique à l'aide du substrat pnp-glucopyranoside. La quantité de paranitrophénol obtenue après action de la beta-glucosidase pourra par exemple être déterminée par lecture de la densité optique à 414 nm.

Un exemple de protocole, que l'homme du métier pourra utiliser pour déterminer si un polypeptide selon l'invention présente une activité enzymatique améliorée par rapport à celle de la protéine sauvage BGL1, est le suivait :
- formation d'une culture stock de *E. coli* exprimant un polypeptide selon l'invention pendant toute la nuit à 37°C ;
- ensemencement d'un milieu de culture LB avec 1% de culture stock pendant 24h à 20°C;
- centrifugation pendant 2 minutes à 13000 rpm ;
- resuspension des culots cellulaires avec du tampon succinate 100 mM à pH 5 (DO₆₀₀ finale 100);
- incubation de 50 µl de cellules avec 100 µl de tampon succinate 100 mM à pH 5 contenant 15 mM de pnp-glucopyranoside pendant 1h30 à 50°C, suivi de 5 minutes sur la glace ;
- ajout de 150 µl de Na₂CO₃ 0.2 M ;
- centrifugation pendant 2 minutes à 13000 rpm ;
- lecture de la densité optique à 414 nm sur 150 µl de surnageant.

De plus, l'homme du métier pourra utiliser le protocole décrit ci-dessus en incubant les 50 µl de cellules avec 100 µl de tampon succinate 100 mM à pH 5 contenant 15 mM de pnp-glucopyranoside et 60 g/L de glucose pendant 1h30 à 50°C, pour déterminer si un polypeptide selon l'invention est moins sensible à l'inhibition par le glucose que la protéine sauvage BGL1.

Dans le cadre de l'invention, un acide aminé dit « altéré » signifie un acide aminé « substitué », « inséré » ou « délété ».

Selon un mode de réalisation, l'acide aminé dit « altéré » est « substitué » par rapport à la séquence d'acides aminés selon SEQ ID No:2.

Selon un mode de réalisation, l'acide aminé dit « altéré » est « inséré » par rapport à la séquence d'acides aminés selon SEQ ID No:2.

Selon un mode de réalisation, l'acide aminé dit « altéré » est « délété » par rapport à la séquence d'acides aminés selon SEQ ID No:2.

Selon un mode de réalisation, le polypeptide tel que décrit précédemment est caractérisé en ce qu'au moins deux acides aminés de la séquence d'acides aminés sont altérés par rapport à la séquence d'acides aminés SEQ ID No :2, lesdits acides aminés altérés étant choisis parmi les positions 225, 238, 240 et 241 de la séquence SEQ ID No :2.

Selon un mode de réalisation, le polypeptide tel que décrit précédemment est caractérisé en ce qu'au moins trois acides aminés de la séquence d'acides aminés sont altérés par rapport à la séquence d'acides aminés SEQ ID No :2, lesdits acides aminés altérés étant choisis parmi les positions 225, 238, 240 et 241 de la séquence d'acides aminés SEQ ID No :2.

Selon un mode de réalisation, le polypeptide tel que décrit précédemment est caractérisé en ce qu'au moins quatre acides aminés de la séquence d'acides aminés sont altérés par rapport à la séquence d'acides aminés SEQ ID No :2, lesdits acides aminés altérés étant ceux des positions 225, 238, 240 et 241 de la séquence d'acides aminés SEQ ID No :2.

Selon un mode de réalisation, le polypeptide tel que décrit précédemment est caractérisé en ce qu'au moins un, au moins deux, au moins trois, ou au moins quatre acides aminés de la séquence d'acides aminés sont altérés par rapport à la séquence d'acides aminés SEQ ID No :2, lesdites altérations étant choisies parmi Q225H, V238I, T240G et T241S.

Selon un mode de réalisation, le polypeptide tel que décrit précédemment est caractérisé en ce qu'un acide aminé est altéré par rapport à la séquence SEQ ID No: 2, ladite altération étant Q225H.

Selon un mode de réalisation, le polypeptide tel que décrit précédemment est caractérisé en ce que trois acides aminés sont altérés par rapport à la séquence SEQ ID No: 2, lesdites altérations étant V238I, T240G et T241S.

Selon un mode de réalisation, le polypeptide tel que décrit précédemment est caractérisé en ce que quatre acides aminés sont altérés par rapport à la séquence SEQ ID No: 2, lesdites altérations étant Q225H, V238I, T240G et T241S.

Selon un mode de réalisation, le polypeptide tel que décrit précédemment comprend en outre au moins un acide aminé altéré supplémentaire choisi parmi les positions 97, 99, 100, 118, 119, 121, 123, 126, 127, 128, 130, 132, 134, 135, 140, 147, 151, 153, 163, 168, 173, 174, 177, 179, 182, 187, 193, 206, 207, 212, 217 et 621 de la séquence d'acides aminés SEQ ID No:2.

Selon un mode de réalisation, le polypeptide tel que décrit précédemment est caractérisé en ce que ledit acide aminé altéré supplémentaire comprend une ou plusieurs altérations sélectionnées dans le groupe consistant en V97I, Y99F, S100G, V118T, N119E, I121M, E123Q, Q126E, F127Y, I128L, E130A, V132A, A134G, S135C, S135V, I140L, P147A, T151I, Q153H, V163T, T168A, G173A, G173S, Q174E, N177E, I179L, V182A, V182N, T187C, L193V, N206D, P207V, L212M, T217L, L621F et L621T.

Il est également décrit ici un polypeptide sélectionné dans le groupe consistant en :
- une séquence d'acides aminés choisie parmi SEQ ID No :4, SEQ ID NO :6, SEQ ID NO :8, SEQ ID NO :10, SEQ ID NO :12; SEQ ID NO :14 ou
- une séquence d'acides aminés SEQ ID NO : X présentant : i) un pourcentage de résidus identiques par rapport à la longueur de SEQ ID NO : 4, 6, 8, 10, 12 ou 14 d'au moins 70% d'identité, préférentiellement 75%, 80%, 85%, 90%, 95%, 98% ou 99% ; ii) un pourcentage de résidus identiques par rapport à la longueur de SEQ ID NO : X d'au moins 70% d'identité, préférentiellement 75%, 80%, 85%, 90%, 95%, 98% ou 99%.

Des variants des séquences d'acides aminés de la présente invention peuvent être préparés par diverses méthodes conventionnelles, telles que la mutagénèse aléatoire, la mutagénèse dirigée, la synthèse de gènes ou le shuffling, en utilisant la totalité ou une partie des séquences nucléotidiques ou peptidiques présentées dans le présent texte. De tels variants comprennent par exemple des délétions et/ou insertions et/ou substitutions de résidus dans la séquence d'acides aminés de l'enzyme. La présente invention concerne tout variant obtenu à partir des séquences présentées dans ce texte dès lors que les variants desdites séquences d'acides aminés conservent une fonction beta-glucosisase améliorée (telle que définie plus haut) par rapport à Bgl-1.

Il est également décrit ici une séquence d'acides aminés, nommée ci-après pour des raisons d'ordre pratique SEQ ID NO : X, qui lorsqu'alignée avec SEQ ID NO: 4, 6, 8, 10, 12 ou 14, comprend:
a) un pourcentage de résidus identiques par rapport à la longueur de SEQ ID NO : 4, 6, 8, 10, 12 ou 14 d'au moins 70% d'identité, préférentiellement 75, 80%, 85%, 90%, 95%, 98% ou 99% ; et
b) un pourcentage de résidus identiques par rapport à la longueur de SEQ ID NO : X d'au moins 70% d'identité, préférentiellement 75, 80%, 85%, 90%, 95%, 98% ou 99%.

Le pourcentage de résidus identiques par rapport à la longueur de SEQ ID NO: 4, 6, 8, 10, 12 ou 14 correspond au nombre de résidus identiques entre SEQ ID NO: X et SEQ ID NO: 4, 6, 8, 10, 12 ou 14 divisé par le nombre de résidus dans SEQ ID NO: 4, 6, 8, 10, 12 ou 14. Lorsqu'en utilisant la base de données GenomeQuest, lesdits pourcentages de résidus identiques par rapport à la longueur de SEQ ID NO: 4, 6, 8, 10, 12 ou 14 correspondent à des pourcentages d'identité d'Interrogation (% id Query), où Interrogation correspond à la séquence SEQ ID NO: 4, 6, 8, 10, 12 ou 14.

Le pourcentage de résidus identiques par rapport à la longueur de SEQ ID NO: X correspond au nombre de résidus identiques entre SEQ ID NO: X et SEQ ID NO: 4, 6, 8, 10, 12 ou 14 divisé par le nombre de résidus dans SEQ ID NO: X. Lorsqu'en utilisant la base de données GenomeQuest, lesdits pourcentages de résidus identiques par rapport à la longueur de SEQ ID NO: X correspondent à des pourcentages d'identité du Sujet (% id Subject), où Sujet correspond à SEQ ID NO : X.

L'invention a également pour objet, un acide nucléique purifié ou isolé codant pour au moins un polypeptide tel que décrit précédemment. Le tableau 1 ci-dessous comprend les identifications des séquences nucléiques et peptidiques pour les gènes Bgl-1, les gènes A et C, ainsi que pour les polypeptides de l'invention.

**TABLEAU 1**

| **Clones** | **Acide nucléique** | **Polypeptide** |
|---|---|---|
| BGL1 (sauvage) | SEQ NO :1 | SEQ NO :2 |
| 10H7 | SEQ NO :3 | SEQ NO :4 |
| 59B8 | SEQ ID NO :5 | SEQ ID NO :6 |
| 164A2 | SEQ ID NO :7 | SEQ ID NO :8 |
| 100B11 | SEQ ID NO :9 | SEQ ID NO :10 |
| 115E1 | SEQ ID NO :11 | SEQ ID NO :12 |
| 149G7 | SEQ ID NO.13 | SED ID NO :14 |
| Gène A | SEQ ID NO.15 | SED ID NO :16 |
| Gène C | SEQ ID NO.17 | SED ID NO :18 |

L'invention porte également sur un vecteur comprenant un acide nucléique tel que décrit précédemment.

Selon l'invention, on entend par « vecteur » toute séquence d'ADN dans laquelle il est possible d'insérer des fragments d'acide nucléique étranger, les vecteurs permettant d'introduire de l'ADN étranger dans une cellule hôte. Des exemples de vecteurs sont les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus.

Selon l'invention, l'acide nucléique tel que décrit précédemment pourra être lié opérationnellement à un promoteur, un terminateur ou toute autre séquence nécessaire à son expression dans la cellule hôte.

Le vecteur selon l'invention pourra également porter un marqueur de sélection. On entend par « marqueur de sélection » un gène dont l'expression confère aux cellules qui le contiennent une caractéristique permettant de les sélectionner. Il s'agit par exemple d'un gène de résistance aux antibiotiques.

L'invention a également pour objet une cellule hôte isolée comprenant soit au moins l'un des polypeptides tels que décrit précédemment, soit au moins l'un des acides nucléiques tels que décrit précédemment ou soit au moins l'un des vecteurs tels que décrit précédemment.

L'homme du métier pourra introduire au moins l'un des polypeptides, au moins l'un des acides nucléiques ou au moins l'un des vecteurs tels que décrit précédemment dans la cellule hôte par des méthodes conventionnelles bien connues. Par exemple, on peut citer le traitement au chlorure de calcium, l'électroporation, l'utilisation d'un pistolet à particules.

Selon un mode de réalisation, l'homme du métier pourra introduire dans la cellule hôte et par des méthodes conventionnelles, plusieurs copies d'un acide nucléique codant un polypeptide ayant une activité beta-glucosidase améliorée selon l'invention.

Selon un mode de réalisation, la cellule hôte isolée telle que décrite précédemment est choisie parmi *Trichoderma, Aspergillus, Neurospora, Humicola, Penicillium, Fusarium, Thermomonospora, Bacillus, Pseudomonas, Escherichia, Clostridium, Cellulomonas, Streptomyces, Yarrowia, Pichia* et *Saccharomyces.*

Selon un mode de réalisation préférée, la cellule hôte isolée telle que décrite précédemment est choisie parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Penicillium pinophilum, Pénicillium oxalicum, Escherichia coli, Clostridium acetobutylicum, Clostridium saccharolyticum, Clostridium benjerinckii, Clostridium butylicum, Pichia pastoris, Yarrowia lipolityca* et leurs mélanges.

Selon un mode de réalisation préférée, la cellule hôte isolée telle que décrite précédemment est *Trichoderma reesei.*

L'invention porte également sur l'utilisation de l'un quelconque des polypeptides décrits précédemment pour l'hydrolyse des beta-oligosaccharides.

L'invention porte également sur l'utilisation de l'un quelconque des polypeptides décrits précédemment pour l'hydrolyse du cellobiose en glucose.

L'invention a également pour objet, l'utilisation de l'un quelconque des polypeptides décrits précédemment pour la production de biocarburant.

Selon l'invention, le terme biocarburant peut être défini comme tout produit issu de la transformation de la biomasse et pouvant être utilisé à des fins énergétiques. D'une part et sans vouloir se limiter, on peut citer à titre d'exemple des biogaz, des produits pouvant être incorporés (éventuellement après transformation ultérieure) à un carburant ou être un carburant à part entière, tels que des alcools (l'éthanol, le butanol et/ou l'isopropanol selon le type d'organisme fermentaire utilisé), des solvants (acétone), des acides (butyrique), des lipides et leurs dérivés (acides gras à courtes ou longues chaînes, esters d'acides gras), ainsi que l'hydrogène.

De manière préférée, le biocarburant selon l'invention est un alcool, par exemple l'éthanol, le butanol et/ou l'isopropanol. Plus préférentiellement, le biocarburant selon l'invention est l'éthanol.

Dans un autre mode de réalisation, le biocarburant est du biogaz.

Outre la production de biocarburant, les polypeptides ayant une activité beta-glucosidase améliorée selon l'invention peuvent également être utilisés dans d'autres types d'applications en catalysant l'hydrolyse de divers substrats, permettant ainsi la libération d'une variété d'arômes. A titre d'exemple, ils peuvent être utilisés afin de libérer des arômes de fruits en catalysant plusieurs glucosides présents à l'intérieur de ces fruits ou encore, ils peuvent hydrolyser les beta-glucosidases monoterphényl de raisins représentant ainsi une source importante d'arômes pour le vin. Par conséquent, les polypeptides ayant une activité beta-glucosidase améliorée selon l'invention peuvent être utilisés dans plusieurs domaines, notamment en parfumerie, en industrie alimentaire, en oenologie, etc.

Les souches de champignons filamenteux, de préférence *Trichoderma,* plus préférentiellement *T. reesei,* capables d'exprimer au moins un polypeptide selon l'invention sont cultivées en fermenteurs, en présence d'un substrat carboné, tel que le lactose ou le glucose, choisi pour la croissance du microorganisme. Dans un mode de réalisation, ce substrat carboné, selon sa nature, est introduit dans le fermenteur avant stérilisation ou est stérilisé séparément et introduit dans le fermenteur après stérilisation de ce dernier pour obtenir une concentration initiale de 20 à 35 g/L.

Une solution aqueuse contenant le substrat choisi pour la production des enzymes est ensuite ajoutée. Une composition enzymatique agissant sur la biomasse lignocellulosique produite par les champignons est enfin récupérée par filtration du milieu de culture. Dans cette composition, on retrouve notamment l'endoglucanase, l'exoglucanase et la beta-glucosidase selon l'invention. Dans un mode de réalisation, la solution aqueuse contenant le substrat choisi pour la production des enzymes est préparée à la concentration de 200-250 g/L ; cette solution doit contenir le substrat inducteur tel que le lactose. Cette solution aqueuse est injectée après l'épuisement du substrat carboné initial de façon à apporter une quantité optimisée, comprise entre 35 et 45 mg/g de cellules ("fed batch"). Pendant cette phase de "fed batch", la concentration résiduelle en sucre dans le milieu de culture est inférieure à 1 g/L et les enzymes agissant sur la biomasse lignocellulosique sont sécrétées par le champignon. Ces dernières peuvent être récupérées par filtration du milieu de culture.

L'invention a pour objet une composition enzymatique agissant sur la biomasse lignocellulosique, ladite composition enzymatique étant produite par des champignons filamenteux et comprenant au moins un polypeptide ayant une activité beta-glucosidase améliorée par rapport à l'activité beta-glucosidase de la protéine sauvage BGL1.

Enfin, l'invention a pour objet un procédé de production de biocarburant à partir de la biomasse comprenant les étapes suivantes :
- mise en suspension en phase aqueuse du matériau à hydrolyser ;
- ajout d'une composition enzymatique agissant sur la biomasse lignocellulosique telle que décrite précédemment;
- dosage des sucres libérés ;
- séparation de la solution sucrée de la fraction solide non hydrolysée ;
- fermentation de la solution sucrée ;
- séparation du biocarburant du moût de fermentation.

Dans un mode de réalisation, le matériau à hydrolyser est mis en suspension en phase aqueuse à raison de 6 à 40 % de matière sèche, de préférence 20 à 30 %. Le pH est ajusté entre 4 et 5,5, de préférence entre 4,8 et 5,2 et la température entre 40 et 60°C, de préférence entre 45 et 50°C. La réaction d'hydrolyse est démarrée par l'ajout de la composition enzymatique agissant sur la biomasse lignocellulosique; la quantité habituellement utilisée est de 10 à 30 mg de protéines excrétées par gramme de substrat prétraité ou moins. La réaction dure généralement de 15 à 48 heures. La réaction est suivie par dosage des sucres libérés, notamment le glucose. La solution sucrée est séparée de la fraction solide non hydrolysée, essentiellement constituée de lignine, par filtration ou centrifugation ; elle est utilisée pour la fermentation.

Dans un mode de réalisation, le biocarburant pourra être séparé du moût de fermentation par distillation.

Un autre objet de l'invention est un procédé de production de biocarburant à partir de la biomasse, caractérisé en ce qu'il comprend les étapes suivantes :
- mise en suspension en phase aqueuse de la biomasse à hydrolyser ;
- ajout simultané d'une composition enzymatique agissant sur la biomasse lignocellulosique telle que définie précédemment et d'un organisme fermentaire;
- séparation du biocarburant du moût de fermentation.

Selon ce mode de réalisation, la cellulose présente dans la biomasse est convertie en glucose, et en même temps, dans le même réacteur, l'organisme fermentaire par exemple une levure) convertit le glucose en produit final selon un procédé de SSF (Simultaneous Saccharification and Fermentation) connu de l'homme du métier. Selon les capacités métaboliques et hydrolytiques de l'organisme fermentaire, le bon déroulement de l'opération peut nécessiter l'addition d'une quantité plus ou moins importante de mélange cellulolytique exogène.

Dans un autre mode de réalisation, un même organisme fermentaire pourra être capable de convertir la biomasse en glucose puis le glucose en produit final.

L'utilisation du polypeptide présentant une meilleure activité beta-glucosidase selon la présente invention présente ainsi l'avantage d'obtenir un meilleur rendement de production de glucose. Ainsi la présente invention permet d'utiliser moins d'enzyme qu'auparavant, ce qui présente un avantage économique, le coût de production du biocarburant, par exemple, étant moindre.

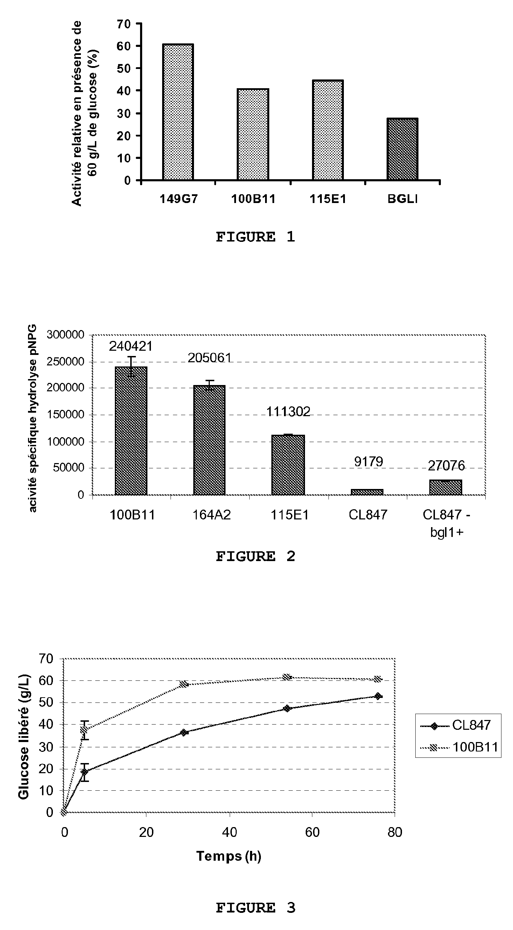

D'autres aspects, objets, avantages et caractéristiques de l'invention, seront présentés à la lecture de la description non restrictive qui suit et qui décrit des modes de réalisation préférés de l'invention donnés par le biais d'exemples et de la figure 1.
La Figure 1 est un graphique représentant l'amélioration de l'activité beta-glucosidase pour les variants 149G7, 100B11, 115E1 en comparaison avec le gène parent BGL1, lorsqu'en présence de glucose.
La Figure 2 est un graphique représentant l'activité spécifique beta-glucosidase des mélanges enzymatiques produits en fiole par les clones transformés (100B11, 164A2 et 115E1) et la souche de départ CL847. La souche CL847-bgl1+ est indiquée à titre comparatif.
La Figure 3 est un graphique représentant les résultats des hydrolyses (glucose libéré) pour les enzymes produites par la souche de référence (CL847) et le variant (100B11).

### EXEMPLES

### EXEMPLE 1 : 1er tour de shuffling

La séquence du gène de la beta-glucosidase de *Trichoderma reesei* (gène parental BGL1, SEQ ID NO : 1) a été soumise à un premier tour de shuffling selon le procédé breveté décrit dans EP 1104457B1 avec le gène de la glucosidase putative de *Chaetomium globosum* (gène A) (SEQ ID NO : 15 et SEQ ID NO : 16 (séquence protéique)) possédant 70% d'identité avec le gène parental BGL1.

### 1- Criblage à haut débit

Un test de criblage à haut débit a permis de sélectionner les meilleurs clones issus du shuffling de ces deux séquences, c'est-à-dire ceux présentant un facteur d'amélioration supérieur à 2 au niveau de l'activité beta-glucosidase lorsque comparés avec le gène parental BGL1 de *T. reesei.*

Le test de criblage de la banque du premier tour de shuffling a été réalisé selon les étapes suivantes :
- isolement sur gélose des différentes colonies de *E.coli* exprimant les variants de shuffling de l'enzyme recombinante selon l'invention et mise en pré-cultures en milieu LB desdites colonies pendant la nuit à 37°C;
- inoculation d'un milieu LB à 3% avec la pré-culture puis incubation 4h à 37°C ;
- induction de l'expression des variants par addition d'isopropyl-beta-thio-galactoside (IPTG) 100 µM puis incubation à 20°C pendant la nuit ;
- centrifugation pendant 2 minutes à 13000 rpm ;
- re-suspension des culots cellulaires dans 100 µL de tampon succinate 0.1M contenant 2.2 mM de para-nitrophenyl-D-glucoside-6-phosphate (pNPGlc) ;
- incubation 3h à température ambiante ;
- lecture de la densité optique à 414 nm après alcalinisation.

Dans ces conditions de criblage, une amélioration de l'activité beta-glucosidase par rapport à l'enzyme de référence BGLI a été trouvée dans plusieurs clones, dont notamment les clones 10H7 (SEQ ID NO :3 et 4), 59B8 (SEQ ID NO : 5 et 6) et 164A2 (SEQ ID NO : 7 et 8).

### 2-Détermination de l'amélioration de l'activité β-glucosidase.

### 2-1/ Sur le substrat pNPGlc

Afin de déterminer le kcat relatif des variants sélectionnés au premier tour de shuffling, on procède de la façon suivante :
- formation d'une culture stock de *E. coli* exprimant une enzyme recombinante selon l'invention pendant toute la nuit à 37°C ;
- ensemencement d'un milieu de culture LB avec 1% de culture stock pendant 24h à 20°C avec induction à l'IPTG (250 µM);
- centrifugation pendant 2 minutes à 13000 rpm ;
- resuspension des culots cellulaires avec du tampon succinate 100 mM à pH 5 (DO₆₀₀ finale 100);
- incubation de 50 µl cellules avec 100 µl de tampon succinate 100 mM à pH 5 contenant 15 mM de pnp-glucopyranoside pendant 1h30 à 50°C suivi de 5 minutes sur la glace ;
- ajout de 150 µl de Na₂CO₃ 0.2 M ;
- centrifugation pendant 2 minutes à 13000 rpm ;
- lecture de la densité optique à 414 nm sur 150 µl de surnageant.

Le tableau 2 présente les valeurs des kcat ainsi que les facteurs d'améliorations obtenus pour les clones 10H7, 59B8 et 164A2 dans ces conditions expérimentales.

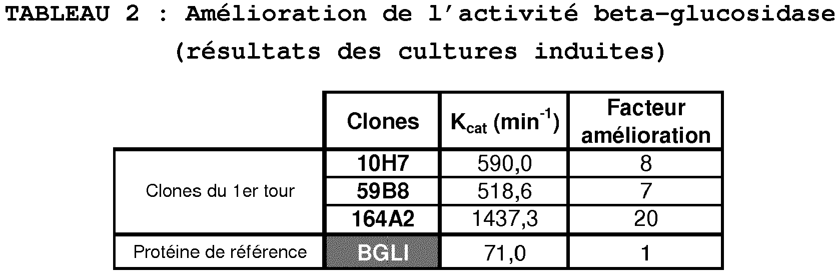

Les résultats montrent des améliorations d'activités enzymatiques très importantes par rapport à l'enzyme de référence (BGLI) pour les 3 clones 10H7, 59B8 et 164A2.

### 2-2/ Sur le cellobiose

L'amélioration d'activité des clones 10H7, 59B8 et 164A2 a ensuite été confirmée sur un second substrat : le cellobiose.

Ce test a été effectué sur des cultures de *E. coli* exprimant une enzyme recombinante selon l'invention. Les étapes du test sont les suivantes :
- Ensemencer un milieu de culture LB avec 1% de culture stock induites avec de l'IPTG puis incubation toute la nuit à 37°C.
- Cultiver lesdites cellules à 37°C jusqu'à l'obtention d'une densité optique à 600 nm de 0.4.
- Induire lesdites cellules avec de l'IPTG 250 µM à 20°C pendant 20 heures.
- Laver les culots cellulaires trois fois dans un tampon succinate 100 mM pH 5 pour éliminer le glucose du milieu de culture.
- Incuber 10 µl desdites cellules avec 190 µl de cellobiose à 263.2 mM (250 mM finale) pendant 12 heures à 50°C en microplaque.

### Révélation :

- Mélanger et incuber pendant 1h à température ambiante
   - 10 µl réaction ci-dessus
   - 90 µl tampon succinate 100 mM à pH 5
   - 5 µl glucose oxydase 44 U/ml
- Mélanger et incuber pendant 30 min à température ambiante
   - 10 µl réaction glucose oxydase
   - 2 µl horse radish peroxydase à 10 U/ml
   - 5 µl ABTS 100 mM
   - 83 µl tampon phosphate 50 mM pH 7.4

Lire les densités optiques à 420 nm.

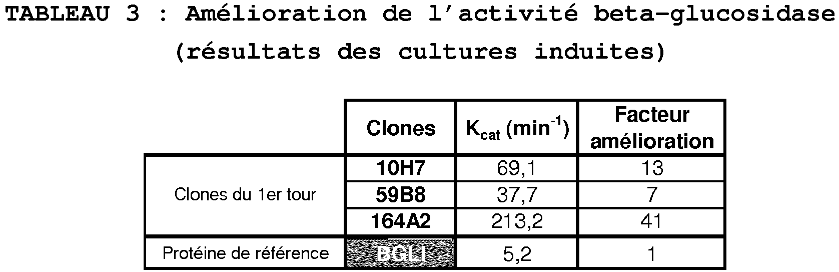

De même, les résultats montrent des améliorations d'activités enzymatiques très importantes par rapport à l'enzyme de départ (BGL1) pour les clones 10H7, 59B8 et 164A2 lorsque le cellobiose est utilisé comme substrat.

### EXEMPLE 2 : 2eme tour de shuffling

Les séquences des gènes améliorés obtenu au premier tour de shuffling ont par la suite été soumises à un deuxième tour de shuffling (toujours selon le procédé breveté décrit dans EP1104457B1). Afin d'augmenter la diversité génétique, au moins un gène codant pour une beta-glucosidase ayant 70% identité a été ajouté. Dans cet exemple précis, le gène de la glucosidase putative de *Neurospora crassa* (gène C) (SEQ ID NO : 17 et SEQ ID NO : 18 (séquence protéique)) a été utilisé.

### 1- Criblage à haut débit

Un test de criblage à haut débit tel que décrit précédemment (à l'exception de l'étape d'induction à l'IPTG, puisque l'amélioration apportée au premier tour de shuffling permet une détection de l'activité beta-glucosidase basée uniquement sur la fuite du promoteur) a été effectué sur les clones obtenus à la suite de ce second tour de shuffling, afin de sélectionner les meilleurs clones, c'est-à-dire ceux présentant un facteur d'amélioration supérieur à 2 au niveau de l'activité beta-glucosidase lorsque comparés avec le clone 164A2.

Dans ces conditions de criblage, une amélioration de l'activité beta-glucosidase par rapport à l'enzyme de référence (164A2) a été trouvée dans plusieurs clones, dont notamment les clones 100B11 (SEQ ID NO :9 et 10), 115E1 (SEQ ID NO : 11 et 12).

### 2-Détermination de l'amélioration de l'activité β-glucosidase.

### 2-1/ Sur le pNPGlc

Afin de déterminer le kcat relatif, les activités des clones 100B11 et 115E1 ont été mesurées par le test d'activité tel que décrit précédemment.

Le tableau 4 présente les valeurs des kcat ainsi que les facteurs d'améliorations obtenus pour les clones 100B11 et 115E1 dans ces conditions expérimentales.

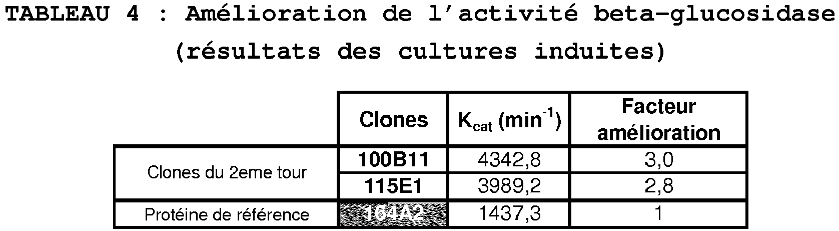

Les résultats montrent des améliorations d'activités enzymatiques très importantes par rapport à l'enzyme de référence (164A2) et à (BGLI) (X60) pour les clones 100B11 et 115E1.

### 2-2/ Sur le cellobiose

L'amélioration d'activité des clones 100B11 et 115E1 a ensuite été confirmée sur un second substrat : le cellobiose.

Afin de déterminer le kcat relatif, les activités des clones 100B11 et 115E1 ont été mesurées par le test d'activité tel que décrit précédemment en utilisant le cellobiose comme substrat.

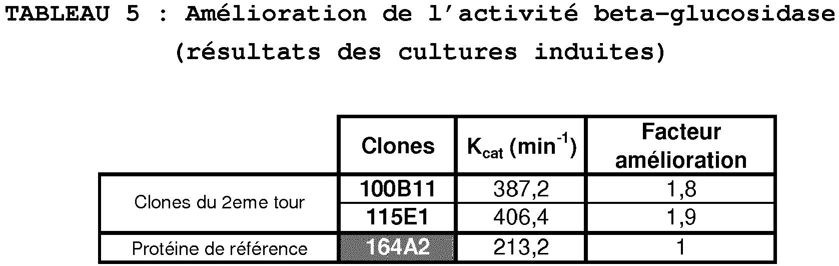

De même, les résultats montrent des améliorations d'activités enzymatiques importantes par rapport à l'enzyme de référence (164A2) pour les clones 100B11 et 115E1 lorsque le cellobiose est utilisé comme substrat.

### EXEMPLE 3 : 3ème tour de shuffling

Les séquences des gènes améliorés obtenu au deuxième tour de shuffling ont par la suite été soumises à un troisième tour de shuffling (toujours selon le procédé breveté décrit dans EP1104457B1). Afin d'augmenter la diversité génétique, au moins un gène codant pour une beta-glucosidase ayant 70% identité a été ajouté. Dans cet exemple précis, le gène de la glucosidase putative de *Neurospora crassa* (gène C) (SEQ ID NO : 17 et SEQ ID NO : 18) et le gène de la glucosidase putative de *Chaetomium globosum* (gène A) (SEQ ID NO : 15 et SEQ ID NO : 16) ont été utilisés.

### 1- Criblage à haut débit

Un test de criblage à haut débit tel que décrit précédemment (à l'exception de l'étape d'induction à l'IPTG, puisque l'amélioration apportée au premier tour de shuffling permet une détection de l'activité beta-glucosidase basée uniquement sur la fuite du promoteur) a été effectué sur les clones obtenus à la suite de ce troisième tour de shuffling, afin de sélectionner les meilleurs clones, c'est-à-dire ceux présentant un facteur d'amélioration supérieur à 2 au niveau de l'activité beta-glucosidase lorsque comparés avec le clone 115E1.

Dans ces conditions de criblage, une amélioration de l'activité beta-glucosidase par rapport à l'enzyme de référence (115E1) a été trouvée notamment pour le clone 149G7 (SEQ ID NO : 13 et 14).

### 2-Détermination de l'amélioration de l'activité β-glucosidase.

### 2-1/ Sur le pNPGlc

Afin de déterminer le kcat relatif, l'activité du clone 149G7 a été mesurée par le test d'activité tel que décrit précédemment.

Les résultats montrent une amélioration de l'activité enzymatique du clone 149G7 de 2,4 fois par rapport au clone 115E1 et de plus de 100 fois par rapport à BGL1.

### EXEMPLE 4 : Activité β-glucosidase améliorée en présence de glucose

Afin de comparer l'activité des clones 149G7, 100B11, 115E1 avec BGLI, l'activité de ces clones a été mesurée par le test d'activité tel que décrit précédemment sur le pNPGlc en présence de 60g/L de glucose dans le milieu réactionnel (produit de la réaction).

La figure 1 montre que le clone 149G7 retient 61% de son activité en présence de 60g/L de glucose alors que la protéine parentale de référence BGLI n'en retient que 27%.

Bien que la présente invention ait été décrite ci-dessus par le biais d'exemples de ses modes de réalisation préférées, il est entendu qu'elle peut être modifiée sans se détourner de l'esprit et de la nature de l'invention telle que définie dans les revendications annexées.

### EXEMPLE 5 : Transformation de T. reesei avec les variants améliorés de beta-glucosidases

Chaque gène correspondant aux variants 115E1, 100B11 et 164A2, a été cloné dans un vecteur permettant l'expression dans *Trichoderma reesei* avec sélection par l'hygromycine. Le gène a été placé sous contrôle d'un promoteur fort *cbh1,* inductible en même temps que les autres cellulases de *T. reesei.*

La transformation de *Trichoderma reesei* a été réalisée selon les méthodes classiques connues de l'homme de l'art (transformation de protoplastes par choc calcique et sélection à l'hygromycine 50µg/mL). Les transformants ont été purifiés par sporulation puis repiqués deux fois en milieu sélectif pour élimination des clones instables. L'intégration de l'ADN d'intérêt a ensuite été contrôlée par PCR selon la méthode décrite par Yu et coll., Fungal Genet. Biol. (2004); 41(11):973-981.

Les clones positifs en PCR ont ensuite été évalués en production de cellulases en fioles. Quelques spores de chaque clone ont été utilisées pour ensemencer 50 ml de milieu PD Broth (Difco). Les fioles ont été mises à incuber 72h à 30°C avec agitation à 150 rpm. Au bout de 72h cette pré-culture a servie à ensemencer à 30% un milieu de production de cellulases ayant la composition suivante: lactose (20g/L), cellulose Solka floc (20g/L), Peptone (5g/L), KH₂PO₄ (15g/L), (NH₄)₂SO₄ (5g/L), CaCl₂ (0,6g/L), MgSO₄ (0,6g/L), FeSO₄ (0,005g/L), MnSO₄ (0,0014g/L), ZnSO₄ (0,0014g/L), CoCl₂ (0,0037 g/L), acide maléique (11,6 g/L), Tris (12,1 g/L) et NaOH (2,08 g/L).

Les cultures ont été mises à incuber à 30°C à 150 tr/min d'agitation. Au bout de 5 jours les cultures ont été centrifugées et la concentration en protéines du surnageant mesurée par la méthode de Bradford. L'activité beta-glucosidase des surnageants a été mesurée par hydrolyse du substrat chromophore p-nitrophenyl beta-D-glucoside (pNPG) dans les conditions suivantes:
- 50 mM de tampon citrate à pH 4,8
- 5 mM de pNPG
- 10 µl d'échantillon
- incubation à 50°C pendant 30 min.

La réaction a été arrêtée par ajout de 100 µL de carbonate de sodium à 2%. La quantité de paranitrophénol libéré par hydrolyse du pNPG a été mesurée par mesure de l'absorbance à 410 nm et comparée à une gamme de paranitrophénol. La réaction était linéaire de 25 à 400 µM de paranitrophénol.

La Figure 2 montre les résultats obtenus pour chaque variant (un exemple par variant), en comparaison avec les activités mesurées avec une souche non transformée (CL847) et avec une souche transformée avec la beta-glucosidase native de *T. reesei* (CL847-bgl1+).

Le Tableau 6 présente les facteurs d'amélioration par rapport aux souches de départ CL847 (Durand et al, Enzyme Microb. Technol., 1988; 10:341-346) et CL847-bgl1+, qui surexprime la beta-glucosidase *bgl1* native de *T. reesei.*

**TABLEAU 6 : Facteurs d'augmentation d'activité spécifique beta-glucosidase par rapport à la référence (données issues de la Figure 2.)**

| **Clones** | **Facteur d'augmentation** |
|---|---|
| CL847 | - |
| CL847-bgl1+ | 2,9 |
| 100B11 | 26,2 |
| 164A2 | 22,3 |
| 115E1 | 12,1 |

### EXEMPLE 6 : Activités enzymatiques d'une composition de cellulases produite en fermenteur par un transformant de T. reesei exprimant une beta-glucosidase améliorée

Le variant 100B11 de l'exemple 5 a été utilisé pour réaliser une production de cellulases en fermenteur de 2L.

La production de cellulases est effectuée en fermenteur agité mécaniquement. Le milieu a la composition suivante : KOH (1,66 g/L), H₃PO₄ 85 % (2 mL/L), (NH₄)₂SO₄ 2,8 (g/L), MgSO₄, 7 H₂O (0,6 g/L), CaCL₂ (0,6 g/L), MnSO₄ 3,2 (mg/L), ZnSO₄, 7 H₂O (2,8 mg/L), CoCl₂ (4,0 mg/L), FeSO₄, 7 H₂O (10 mg/L), Corn Steep (1,2 g/L), anti-mousse (0,5 mL/L).

Le fermenteur contenant 1,75 L de milieu minéral et 70g de lactose est stérilisé à 120 °C, puis ensemencé avec 0,25 L d'une pré-culture liquide de la souche de *Trichoderma reesei* CL847. Le milieu de la pré-culture, additionné de phtalate de potassium à 5 g/L pour contrôler les variations de pH, est identique à celui du fermenteur. La croissance du champignon en pré-culture est faite sur lactose, à la concentration de 30 g/L. La croissance de l'inoculum dure de 2 à 3 jours et est effectuée entre 27 et 30°C sur une table d'agitation.

Après 46 heures de croissance, le substrat initial du fermenteur est épuisé et la solution de lactose à 250 g/L est injectée en continu au débit de 4,5 mL/h jusqu'à 142 heures.

La température est régulée à 27°C pendant la phase de croissance de la biomasse, puis à 25°C jusqu'à la fin de la culture. Le pH est régulé à 5 pendant la phase de croissance, puis à 4 jusqu'à la fin de la culture par addition d'une solution d'ammoniaque qui apporte l'azote nécessaire à la synthèse des protéines excrétées. La teneur en oxygène dissous est maintenue au-dessus de 15 à 20 % par action sur l'aération et l'agitation.

La production d'enzymes est suivie par le dosage des protéines extracellulaires par la méthode de Folin (Lowry, Biol. Chem. 1951; 193:265-275), après séparation du mycélium par filtration ou centrifugation. L'activité beta-glucosidase a été mesurée selon la méthode décrite ci-dessus (voir exemple 5) avec le substrat pNPG. L'activité papier-filtre FPase, a été mesurée selon la méthode recommandée par Mandels et coll. Biotechnology for Biofuels, 2009; 2:21. Les résultats sont présentés au Tableau 7.

**TABLEAU 7**

| | **Souches utilisées pour la production** | | |
|---|---|---|---|
| | **CL847** | **CL847-bgl1+** | **100B11** |
| Activité beta-glucosidase spécifique (IU/mg) du mélange cellulolytique produit | 10055 | 45176 | 119677 |
| Activité FPase spécifique (IU/mg) du mélange cellulolytique produit | 0, 5 | 0,4 | 0,65 |
| Facteur d'augmentation de l'activité beta-glucosidase par rapport à la souche CL847 | - | 4, 5 | 11,9 |

### EXEMPLE 7 : Efficacité hydrolytique sur substrat lignocellulosique prétraité du mélange cellulolytique produit par un transformant de T. reesei exprimant une beta-glucosidase améliorée

Les mélanges cellulolytiques produits par la souche de référence CL847 et par le transformant exprimant la beta-glucosidase améliorée 100B11 issus de l'exemple 6 ont été utilisées pour hydrolyser de la paille de blé prétraitée par explosion à la vapeur en conditions acides. Les hydrolyses sont réalisées dans un bioréacteur Bio-Laffite à double enveloppe, agité avec deux agitateurs de type "ancre de marine" dans les conditions expérimentales suivantes:
- substrat lignocellulosique dilué à 10% de matière sèche
- volume réactionnel 2L
- Tampon acétate 1M pH 4,8 (pH contrôlé quotidiennement)
- température 48°C

L'ensemble est imprégné 12h à 300 tr/min avant ajout de 20 mg/g de matière sèche d'enzyme et passage à 500 tr/min. Des prélèvements sont effectués à 0h, 5h, 24h, 48h et 72h après l'ajout des enzymes. Les enzymes sont inactivés par passage de l'échantillon 10 minutes au bain-marie bouillant. L'échantillon est ensuite centrifugé et le surnageant filtré avant dosage du glucose en HPLC.

Les résultats sont présentés en Figure 3. Dès 24h, le mélange enzymatique contenant la beta-glucosidase améliorée produit par le transformant 100B11 a libéré deux fois plus de glucose que le mélange enzymatique produit par la souche de référence (CL847). Le rendement maximal est atteint dès 24h pour le mélange issu de la souche 100B11. A 72h ce rendement n'est toujours pas atteint pour le mélange enzymatique de référence (CL847).

Le mélange enzymatique produit par le transformant 100B11 présente donc une efficacité nettement supérieure à celle du mélange enzymatique de référence (CL847) pour une même dose d'enzyme. Cette propriété se traduit par un rendement et une productivité supérieurs permettant une hydrolyse plus complète du substrat. Alternativement elle permettrait de diminuer la dose d'enzyme à utiliser pour obtenir un résultat équivalent en hydrolyse. Le coût des enzymes cellulolytiques représentant une partie importante du prix de revient du bioéthanol lignocellulosique, toute diminution significative de la quantité d'enzymes à utiliser peut être considérée comme une amélioration importante du procédé.

## Revendications

1. Polypeptide isolé ou purifié ayant une activité beta-glucosidase améliorée par rapport à l'activité beta-glucosidase de la protéine sauvage BGL1 (SEQ ID NO :2), **caractérisé en ce qu'**il comprend une séquence d'acides aminés dans laquelle au moins un acide aminé est altéré par rapport à la séquence d'acides aminés SEQ ID No :2, ledit acide aminé altéré étant choisi parmi les positions 225, 238, 240 et 241 de la séquence d'acides aminés SEQ ID No :2 et
**caractérisé en ce que** ledit polypeptide est choisi dans le groupe constitué de
- une séquence d'acides aminés choisie parmi SEQ ID No :4, SEQ ID NO :6, SEQ ID NO :8, SEQ ID NO :10, SEQ ID NO :12; SEQ ID NO :14;
- une séquence d'acides aminés ayant au moins 90% d'identité, préférentiellement 95%, 98% ou 99% avec la séquence SEQ ID N°2 et comprenant l'altération Q225H;
- une séquence d'acides aminés ayant au moins 90% d'identité, préférentiellement 95%, 98% ou 99% avec la séquence SEQ ID N°2 et comprenant les altérations V238I, T240G et T241S; et
- une séquence d'acides aminés ayant au moins 90% d'identité, préférentiellement 95%, 98% ou 99% avec la séquence SEQ ID N°2 et comprenant les altérations Q225H, V238I, T240G et T241S.

2. Polypeptide selon la revendication **1, caractérisé en ce que** ledit polypeptide comprend en outre au moins un acide aminé altéré supplémentaire choisi parmi les positions 97, 99, 100, 118, 119, 121, 123, 126, 127, 128, 130, 132, 134, 135, 140, 147, 151, 153, 163, 168, 173, 174, 177, 179, 182, 187, 193, 206, 207, 212, 217 et 621 de la séquence d'acides aminés SEQ ID No :2.

3. Polypeptide selon la revendication 1 ou 2, **caractérisé en ce que** ledit polypeptide comprend en outre au moins un acide aminé altéré supplémentaire, ladite altération étant sélectionnée dans le groupe consistant en V97I, Y99F, S100G, V118T, N119E, I121M, E123Q, Q126E, F127Y, I128L, E130A, V132A, A134G, S135C, S135V, I140L, P147A, T151I, Q153H, V163T, T168A, G173A, G173S, Q174E, N177E, I179L, V182A, V182N, T187C, L193V, N206D, P207V, L212M, T217L, L621F et L621T.

4. Acide nucléique purifié ou isolé, **caractérisé en ce qu'**il code pour au moins un polypeptide selon l'une quelconque des revendications **1 à 3.**

5. Vecteur **caractérisé en ce qu'**il comprend un acide nucléique selon la revendication **4.**

6. Cellule hôte isolée **caractérisée en ce qu'**elle comprend soit au moins l'un des polypeptides selon l'une quelconque des revendications **1 à 3,** soit au moins l'un des acides nucléiques selon la revendication **4** ou soit au moins l'un des vecteurs selon la revendication **5.**

7. Cellule hôte isolée selon la revendication **6, caractérisée en ce qu'**elle est choisie parmi *Trichoderma, Aspergillus, Neurospora, Humicola, Penicillium, Fusarium, Thermomonospora, Bacillus, Pseudomonas, Escherichia, Clostridium, Cellulomonas, Streptomyces, Yarrowia, Pichia* et *Saccharomyces.*

8. Cellule hôte isolée selon la revendication **6 ou 7, caractérisée en ce qu'**elle est choisi parmi *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum* et leurs mélanges.

9. Utilisation d'un polypeptide selon l'une quelconque des revendications **1 à 3** pour l'hydrolyse des beta-oligosaccharides.

10. Utilisation d'un polypeptide selon l'une quelconque des revendications **1 à 3** pour l'hydrolyse du cellobiose en glucose.

11. Utilisation d'un polypeptide selon l'une quelconque des revendications **1 à 3** pour la production de biocarburant.

12. Composition enzymatique agissant sur la biomasse lignocellulosique, ladite composition enzymatique étant produite par des champignons filamenteux et comprenant au moins un polypeptide selon l'une quelconque des revendications **1 à 3.**

13. Procédé de production de biocarburant à partir de la biomasse, **caractérisé en ce qu'**il comprend les étapes suivantes :
- mise en suspension en phase aqueuse de la biomasse à hydrolyser ;
- ajout d'une composition enzymatique agissant sur la biomasse lignocellulosique selon la revendication **12** pour débuter l'hydrolyse ;
- dosage des sucres libérés ;
- séparation de la solution sucrée de la fraction solide non hydrolysée ;
- fermentation de la solution sucrée ;
- séparation du biocarburant du moût de fermentation.

14. Procédé de production de biocarburant à partir de la biomasse, **caractérisé en ce qu'**il comprend les étapes suivantes :
- mise en suspension en phase aqueuse de la biomasse à hydrolyser ;
- ajout simultané d'une composition enzymatique agissant sur la biomasse lignocellulosique selon la revendication **12** et d'un organisme fermentaire;
- séparation du biocarburant du moût de fermentation.

## Patentansprüche

1. Isoliertes oder gereinigtes Polypeptid mit einer verbesserten Beta-Glucosidase Aktivität im Vergleich zur Beta-Glucosidase Aktivität des Wilddtypproteins BGL1 (SEQ ID NO 2), **dadurch gekennzeichnet, dass** es eine Aminosäuresequenz umfasst, bei welcher wenigstens eine Aminosäure im Vergleich zur Aminosäuresequenz SEQ ID NO 2 verändert ist, wobei die veränderte Aminosäure aus den Positionen 225, 238, 240 und 241 der Aminosäuresequenz SEQ ID NO 2 gewählt ist, und
**dadurch gekennzeichnet, dass** das Polypeptid gewählt ist aus der Gruppe, umfassend
- eine Aminosäuresequenz gewählt aus SEQ ID NO 4, SEQ ID NO 6, SEQ ID NO 8, SEQ ID NO 10, SEQ ID NO 12, SEQ ID NO 14;
- eine Aminosäuresequenz mit wenigstens 90%, vorzugsweise 95%, 98% oder 99% Identität mit der Sequenz SEQ ID NO 2 und umfassend die Veränderung Q225H;
- eine Aminosäuresequenz mit wenigstens 90%, vorzugsweise 95%, 98% oder 99% Identität mit der Sequenz SEQ ID NO 2 und umfassend die Veränderungen V238I, T240G und T241S; und
- eine Aminosäuresequenz mit wenigstens 90%, vorzugsweise 95%, 98% oder 99% Identität mit der Sequenz SEQ ID NO 2 und umfassend die Veränderungen Q225H, V238I, T240G und T241S.

2. Polypeptid nach Anspruch 1, **dadurch gekennzeichnet, dass** das Polypeptid ferner wenigstens eine zusätzliche veränderte Aminosäure umfasst, gewählt aus den Positionen 97, 99, 100, 118, 119, 121, 123, 126, 127, 128, 130, 132, 134, 135, 140, 147, 151, 153, 163, 168, 173, 174, 177, 179, 182, 187, 193, 206, 207, 212, 217 und 621 der Aminosäuresequenz SEQ ID NO 2.

3. Polypeptid nach Anspruch 1 oder 2, **dadurch gekennzeichnet, dass** das Polypeptid ferner wenigstens eine zusätzliche veränderte Aminosäure umfasst, wobei die Veränderung gewählt ist aus der Gruppe, umfassend V97I, Y99F, S100G, V118T, N119E, I121M, E123Q, Q126E, F127Y, I128L, E130A, V132A, A134G, S135C, S135V, I140L, P147A, T151I, Q153H, V163T, T168A, G173A, G173S, Q174E, N177E, I179L, V182A, V182N, T187C, L193V, N206D, P207V, L212M, T217L, L621F und L621 T.

4. Gereinigte oder isolierte Nucleinsäure, **dadurch gekennzeichnet, dass** sie für wenigstens ein Polypeptid nach einem der Ansprüche **1 bis 3** kodiert.

5. Vektor, **dadurch gekennzeichnet, dass** er eine Nucleinsäure nach Anspruch **4** umfasst.

6. Isolierte Wirtszelle, **dadurch gekennzeichnet, dass** sie entweder wenigstens eines der Polypeptide nach einem der Ansprüche **1 bis 3** umfasst, oder wenigstens eine der Nucleinsäuren nach Anspruch **4** oder wenigstens einen der Vektoren nach Anspruch **5.**

7. Isolierte Wirtszelle nach Anspruch **6, dadurch gekennzeichnet, dass** sie gewählt ist aus *Trichoderma*, *Aspergillus*, *Neurospora, Humicola, Penicillium*, *Fusarium*, *Thermomonospora*, *Bacillus*, *Pseudomonas*, *Escherichia*, *Clostridium*, *Cellulomonas, Sti°eptornayces, Yarrowia*, *Pichia* und *Saccharomyces*.

8. Isolierte Wirtszelle nach Anspruch **6 oder 7, dadurch gekennzeichnet, dass** sie gewählt ist aus *Trichoderma reesei*, *Trichoderma viridae*, *Trichoderma koningii*, *Aspergillus niger, Aspergillus nidulans*, *Aspergillus wentii, Aspergillus oryzae, Aspergillus phoenicis, Neurospora crassa, Humicola grisae*, *Penicilliatrna pinophilum, Penicillium oxalicum*, *Escherichia coli, Clostridium acetobutylicum*, *Clostridium saccharolyticum*, *Clostridium benjerinckii*, *Clostridium butylicum* und ihren Mischungen.

9. Verwendung eines Polypeptids nach einem der Ansprüche **1 bis 3** für die Hydrolyse der Beta-Oligosaccharide.

10. Verwendung eines Polypeptids nach einem der Ansprüche **1 bis 3** für die Hydrolyse von Glucose-Cellobiose.

11. Verwendung eines Polypeptids nach einem der Ansprüche **1 bis 3** für die Produktion von Biokraftstoff.

12. Enzymatische Zusammensetzung, die auf Lignocellulose-Biomasse wirkt, wobei die enzymatische Zusammensetzung durch filamentöse Pilze hergestellt wird und wenigstens ein Polypeptid nach einem der Ansprüche **1 bis 3** umfasst.

13. Verfahren zur Herstellung von Biokraftstoff aus Biomasse, **dadurch gekennzeichnet, dass** es die folgenden Schritte umfasst:
- Suspendieren der zu hydrolysierenden Biomasse in einer wässrigen Phase;
- Hinzufügen einer auf die Lignocellulose-Biomasse wirkenden enzymatischen Zusammensetzung nach Anspruch **12** zum Starten der Hydrolyse;
- Dosierung der freigesetzten Zucker;
- Trennen der Zuckerlösung von dem nicht hydrolysierten Festanteil;
- Fermentierung der Zuckerlösung;
- Trennen des Biokraftstoffes von dem Fermentierungs-Most.

14. Verfahren zur Herstellung von Biokraftstoff aus Biomasse, **dadurch gekennzeichnet, dass** es die folgenden Schritte umfasst:
- Suspendieren der zu hydrolysierenden Biomasse in einer wässrigen Phase;
- gleichzeitiges Hinzufügen einer auf die Lignocellulose-Biomasse wirkenden enzymatischen Zusammensetzung nach Anspruch **12** und eines Fermentierungs-Organismus;
- Trennen des Biokraftstoffes von dem Fermentierungs-Most.

## Claims

1. Isolated or purified polypeptide having an improved beta-glucosidase activity compared with the beta-glucosidase activity of the wild-type BGL1 protein (SEQ ID No. 2), **characterized in that** it comprises an amino acid sequence in which at least one amino acid is modified compared with the amino acid sequence SEQ ID No. 2, said modified amino acid being chosen from positions 225, 238, 240 and 241 of the amino acid sequence SEQ ID No. 2, and
**characterized in that** said polypeptide is chosen from the group consisting of
- an amino acid sequence chosen from SEQ ID No. 4, SEQ ID No. 6, SEQ ID No. 8, SEQ ID No. 10, SEQ ID No. 12 and SEQ ID No. 14;
- an amino acid sequence having at least 90% identity, preferentially 95%, 98% or 99%, with the sequence SEQ ID No. 2 and comprising the modification Q225H;
- an amino acid sequence having at least 90% identity, preferentially 95%, 98% or 99%, with the sequence SEQ ID No. 2 and comprising the modifications V238I, T240G and T241S; and
- an amino acid sequence having at least 90% identity, preferentially 95%, 98% or 99%, with the sequence SEQ ID No. 2 and comprising the modifications Q225H, V238I, T240G and T241S.

2. Polypeptide according to Claim 1, **characterized in that** said polypeptide also comprises at least one additional modified amino acid chosen from positions 97, 99, 100, 118, 119, 121, 123, 126, 127, 128, 130, 132, 134, 135, 140, 147, 151, 153, 163, 168, 173, 174, 177, 179, 182, 187, 193, 206, 207, 212, 217 and 621 of the amino acid sequence SEQ ID No. 2.

3. Polypeptide according to Claim 1 or 2, **characterized in that** said polypeptide also comprises at least one additional modified amino acid, said modification being selected from the group consisting of V97I, Y99F, S100G, V118T, N119E, I121M, E123Q, Q126E, F127Y, I128L, E130A, V132A, A134G, S135C, S135V, I140L, P147A, T151I, Q153H, V163T, T168A, G173A, G173S, Q174E, N177E, I179L, V182A, V182N, T187C, L193V, N206D, P207V, L212M, T217L, L621F and L621T.

4. Purified or isolated nucleic acid, **characterized in that** it encodes at least one polypeptide according to any one of Claims 1 to 3.

5. Vector, **characterized in that** it comprises a nucleic acid according to Claim 4.

6. Isolated host cell, **characterized in that** it comprises either at least one of the polypeptides according to any one of Claims 1 to 3, or at least one of the nucleic acids according to Claim 4, or at least one of the vectors according to Claim 5.

7. Isolated host cell according to Claim 6, **characterized in that** it is chosen from *Trichoderma, Aspergillus, Neurospora, Humicola, Penicillium, Fusarium, Thermomonospora, Bacillus, Pseudomonas, Escherichia, Clostridium, Cellulomonas, Streptomyces, Yarrowia, Pichia* and *Saccharomyces.*

8. Isolated host cell according to Claim 6 or 7, **characterized in that** it is chosen from *Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisae, Penicillium pinophilum, Penicillium oxalicum, Escherichia coli, Clostridium acetobutylicum, Clostridium saccharolyticum, Clostridium benjerinckii, Clostridium butylicum* and mixtures thereof.

9. Use of a polypeptide according to any one of Claims 1 to 3, for the hydrolysis of beta-oligosaccharides.

10. Use of a polypeptide according to any one of Claims 1 to 3, for the hydrolysis of cellobiose to glucose.

11. Use of a polypeptide according to any one of Claims 1 to 3, for the production of biofuel.

12. Enzymatic composition which acts on lignocellulosic biomass, said enzymatic composition being produced by filamentous fungi and comprising at least one polypeptide according to any one of Claims 1 to 3.

13. Method for producing biofuel from biomass, **characterized in that** it comprises the following steps:
- suspending in an aqueous phase the biomass to be hydrolyzed;
- adding an enzymatic composition which acts on lignocellulosic biomass, according to Claim 12, in order to begin the hydrolysis;
- assaying the sugars released;
- separating the sugar solution from the nonhydrolyzed solid fraction;
- fermenting the sugar solution;
- separating the biofuel from the fermentation must.

14. Method for producing biofuel from biomass, **characterized in that** it comprises the following steps:
- suspending in an aqueous phase the biomass to be hydrolyzed;
- simultaneously adding an enzymatic composition which acts on lignocellulosic biomass, according to Claim 12, and a fermentative organism;
- separating the biofuel from the fermentation must.
